# EUROPEAN PATENT SPECIFICATION

(11) **EP 2 728 542 B1**
(45) Date of publication and mention of the grant of the patent: **20.02.2019**
(21) Application number: 12804354.4
(22) Date of filing: 29.06.2012
(51) Int. Cl.: A23L 33/00

(54) **FOOD PRODUCT DEVELOPMENT ASSISTANCE DEVICE, FOOD PRODUCT DEVELOPMENT METHOD, FOOD PRODUCT PRODUCTION METHOD, DIETARY EDUCATION ASSISTANCE DEVICE, AND DIETARY EDUCATION METHOD**
UNTERSTÜTZUNGSVORRICHTUNG FÜR NAHRUNGSMITTELENTWICKLUNG, NAHRUNGSMITTELENTWICKLUNGSVERFAHREN, NAHRUNGSMITTELHERSTELLUNGSVERFAHREN, UNTERSTÜTZUNGSVORRICHTUNG FÜR ERNÄHRUNGSKUNDE UND VERFAHREN FÜR ERNÄHRUNGSKUNDE
DISPOSITIF D'AIDE AU DÉVELOPPEMENT D'UN PRODUIT ALIMENTAIRE, PROCÉDÉ DE DÉVELOPPEMENT D'UN PRODUIT ALIMENTAIRE, PROCÉDÉ DE PRODUCTION D'UN PRODUIT ALIMENTAIRE, DISPOSITIF D'AIDE À L'ÉDUCATION ALIMENTAIRE ET PROCÉDÉ D'ÉDUCATION ALIMENTAIRE

(30) Priority: 30.06.2011 JP 2011146781
(43) Date of publication of application: 07.05.2014
(73) Proprietor: Meiji Co., Ltd., Chuo-ku Tokyo 104-8306 (JP)
(72) Inventor: KAMIYA,Tetsu, Odawara-shi Kanagawa 250-0862 (JP)
(74) Representative: Goddar, Heinz J.
(86) International application number: PCT/JP2012/066706
(87) International publication number: WO 2013/002373

(56) References cited:
- WO-A1-2005/117617
- JP-A- 2008 500 822
- HIROSHI MIZUNUMA: 'Enka Shokkai no Ryudo Kaiseki to sono Oyo' NAGARE 27 September 2005, XP008171819 Retrieved from the Internet: <URL:http://www.jsme-fed.org/news letters/2005 9/no3.html> [retrieved on 2012-07-26]
- 'FTR Consulting Menu Ryushiho Software' FUJI TECHNICAL RESEARCH 18 January 2009, XP008171820 Retrieved from the Internet: <URL:http://web.archive.org/ web/20090118221849/http://ftr.co.jp/consult ing3 /0301 3 fr.html> [retrieved on 2012-07-26]
- MASAMITSU HARUNA: 'Rinshoi Kogaku Skill Up Koza' OSAKA UNIVERSITY PRESS, SEIICHI WASHIDA 19 March 2010, pages 160 - 164, XP008172182
- H. Mizunuma ET AL: "NUMERICAL MODELING AND SIMULATION ON THE SWALLOWING OF JELLY", Journal of Texture Studies, vol. 40, no. 4, 1 August 2009 (2009-08-01) , pages 406-426, XP055200635, ISSN: 0022-4901, DOI: 10.1111/j.1745-4603.2009.00189.x
- Jenny Bloomfield ET AL: "Mathematical modelling of the normal swallow", , 12 November 2010 (2010-11-12), XP055200637, Retrieved from the Internet: URL:http://www.maths-in-medicine.org/uk/20 10/swallowing/report.pdf
- NICOSIA ET AL: "A planar finite element model of bolus containment in the oral cavity", COMPUTERS IN BIOLOGY AND MEDICINE, NEW YORK, NY, US, vol. 37, no. 10, 8 August 2007 (2007-08-08), pages 1472-1478, XP022189616, ISSN: 0010-4825, DOI: 10.1016/J.COMPBIOMED.2007.01.007

## Description

### Technical Field

The present invention relates to a food product development assistance apparatus, a food product development method, a food product production method, a dietary education assistance apparatus, and a dietary education method. More specifically, the present invention relates to a food product development assistance apparatus, a food product development method, a food product production method, a dietary education assistance apparatus, and a dietary education method using a swallowing simulation method that analyzes behavior of a fluid or a bolus passing through oral cavity and throat using a particle method.

### Background Art

The swallowing action, in particular, the physical property of the food product and the movements of the oral cavity organ during swallowing, is complicated. Therefore, it is extremely difficult to grasp the phenomenon itself accurately. However, in the fields of medical treatment and nursing, to prevent accidental swallowing and accidental ingestion by an old person and a handicapped person, reductions in risks of accidental swallowing and accidental ingestion have been strived through repetition of various trials and errors. Given that recently there have been accident of choking on konjac jelly, in general food products, it is required to assure safety of a food product using an objective value and index.

Two methods are available for solution of the swallowing phenomenon: a method that directly obtains biological information such as a videofluoroscopic swallowing or a myoelectric potential measurement and a method that indirectly obtains information using, for example, a swallowing robot or a numerical value simulation.

FIG. 20 illustrates exemplary videofluoroscopic swallowing (images taken by X-ray). In the left diagram, liquid 49 is in an oral cavity. In the middle diagram, the liquid 49 partially flows to a throat. In the right diagram, the liquid 49 has been swallowed and disappeared.

FIG. 21 illustrates an exemplary myoelectric potential measurement. Electrodes are attached to a masseter and a suprahyoid muscle group to measure a myoelectric potential waveform. Then, the myoelectric potential waveform is integrated to calculate a muscle activity amount.

Although the method that directly obtains biological information allows grasping a behavior during swallowing accurately, in gathering data under various conditions, there is a disadvantage that a considerable load is taken to an examinee.

Meanwhile, one method of indirectly obtaining the information is to use the swallowing robot (see Kobayashi, et al., Conference on Robotics and Mechatronics Conference Digest, 2005, 117). The swallowing robot is very useful for understanding of simple principle of the swallowing phenomenon. However, a behavior and a structure of each of the oral cavity organs of the robot is not easily changed.

Up to the present, numerical analyses on a behavior of a fluid or a bolus such as a solid material in a living body have been performed. For the fluid, an inside of an analysis target region is separated by a grid referred to as a mesh. Calculations have been performed using a lattice method that analyzes physical quantities (speed, temperature, pressure) at the grid point and the inside of the grid (see Kamizu, et al., The Society of Chemical Engineers 41st Autumn Meeting Presentation Abstracts, 2009, P09). In the case of treating the bolus as a semisolid, calculations have been performed using a structural analysis method for machine components such as a finite element method (see Mizunuma, et al., The Japan Society of Mechanical Engineers Annual Conference Proceedings, 2005(2), 83-84).

WO 2005/117617 A1 discloses a diagnostic method for the clinical management of dysphagia. A ST index for determining the physical characteristics of foods is calculated based on measurements performed by a texture meter. Firmness, adhesiveness, springiness and cohesiveness are derived from evaluation results. The method is comprised of administering a portion of food to an individual and measuring the capacity of swallowing.

Mizunuma et al., "Numerical Modeling and Simulation on the Swallowing of Jelly", Journal of Texture Studies, Volume 40, Issue 4, pp. 406-426, 2009 describes a numerical model to simulate the swallowing of a simple jelly bolus. The structure of the pharynx is modeled using a finite element method, and the swallowing movements are defined by pharynx posterior wall shift, laryngeal elevation and epiglottis retroflexion. The rheological characteristics of the jelly is investigated using an oscillatory rheometer and a compression test. A Maxwell three-element model is applied to the rheological model of the jelly. The model constants are obtained from compression tests because the mode of deformation and the stress level of the compression tests were similar to those of the swallowed jelly. The frictional relationship between the organs and the jelly is estimated experimentally from some frictional measurements between the jelly and a wet sloping surface. The results of the simulations for the soft and hard jellies showed different patterns of swallowing that depended on their hardness, and the soft jelly produced faster swallowing because of its flexibility.

### Disclosure of Invention

### Problem to be solved by the Invention

However, with the lattice method, which is a mainstream of the conventional numerical analysis, phenomena such as a large deformation of a surface and a spraying seen at the fluid or the bolus while actually being swallowed is difficult to be caught. Accordingly, reproduction of the actual phenomenon has been difficult. Accordingly, the inventors of the present invention have proposed a swallowing simulation apparatus and a swallowing simulation method, please see WO 2013/002374 A1 (or EP2727529 A1). The swallowing simulation apparatus and the swallowing simulation method facilitate approximate reproduction of actual phenomenon of swallowing.

An object of the present invention is to develop an easy-to-eat or easy-to-drink food product by analyzing a swallowing phenomenon using the swallowing simulation method.

### Means for Solving the Problem

The invention is defined by the independent claims. Advantageous embodiments are subject to the dependent claims.

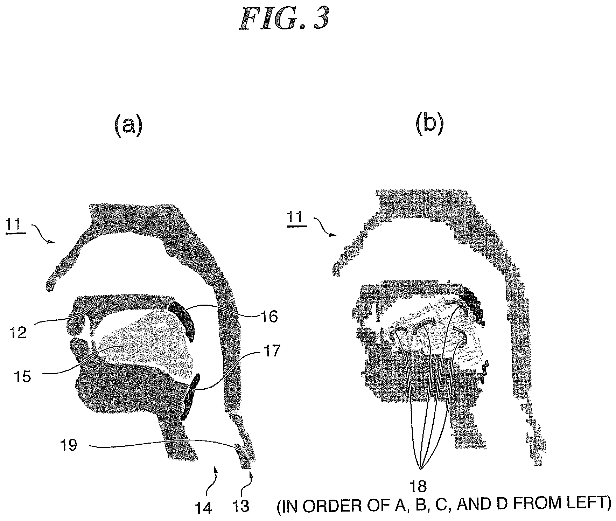

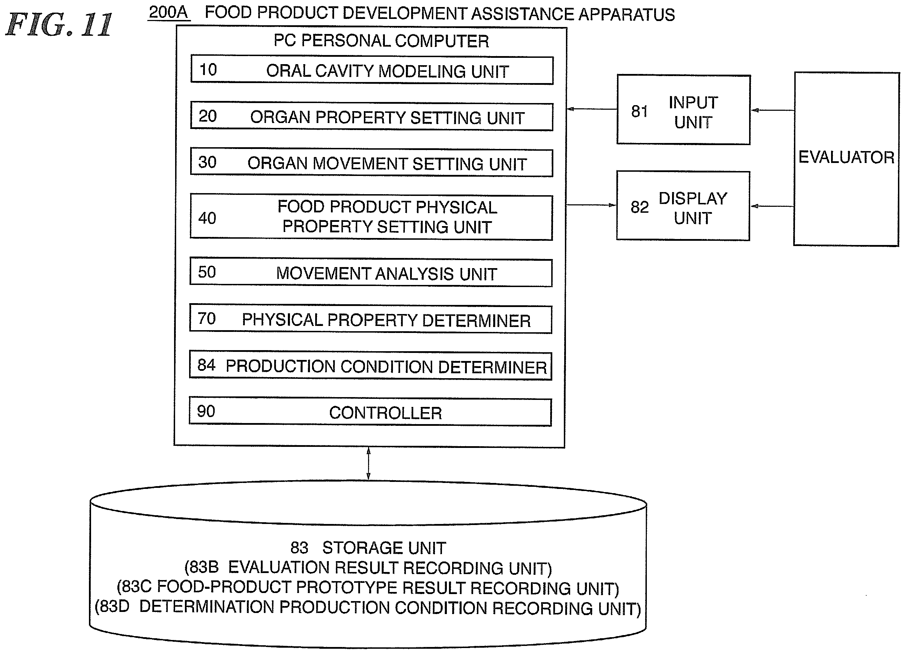

In line with the invention, a food product development assistance apparatus 200A according to the first aspect of the present invention comprises, as shown in, for example, FIG. 11; an oral cavity modeling unit 10 configured to form an oral cavity model 11 (see FIG. 3) formed of oral cavity organs; an organ property setting unit 20 configured to
set an organ property of each of the oral cavity organs in the oral cavity model 11; an organ movement setting unit 30 configured to set a movement of each of the oral cavity organs in the oral cavity model 11; a food product physical property setting unit 40 configured to set a food product, a medicinal product, or a quasi-drug (hereinafter referred to as food product or similar product) as an analysis target, and a physical property of the food product or similar product; an input unit 81 configured to input a pseudo food product to the oral cavity, the pseudo food product being formed by modeling the food product or similar product; a movement analysis unit 50 configured to analyze a movement of each of the oral cavity organs and a behavior of the pseudo food product 41 while being swallowed in the oral cavity model 11 using a particle method; a display unit 82 configured to display an analysis result of the movement of each of the oral cavity organs and the behavior of the pseudo food product 41 while being swallowed on a moving screen, the analysis result being analyzed by the movement analysis unit 50; an evaluation result recording unit 83B configured to record an evaluation result of easiness of eating or easiness of drinking of a food product or similar product based on an analysis result of the behavior of the pseudo food product 41 while being swallowed; a physical property determiner 70 configured to determine the physical property of the food product or similar product regarded as appropriate based on the evaluation result recorded in the evaluation result recording unit 83B; a food product prototype result recording unit 83C configured to record a result of an experimental production performed under an appropriately set production condition to have the physical property determined as appropriate by the physical property determiner 70; and a production condition determiner 84 configured to determine a production condition that sets a physical property of the food product or similar product to the physical property determined as appropriate by the physical property determiner 70 based on a prototype result recorded in the food product prototype result recording unit 83C.

Here, the oral cavity organs are constituted by, an oral cavity wall 12, a gullet 13, a respiratory tract 14, a tongue 15, a soft palate 16, an epiglottis 17 and the like (see FIG. 3). The oral cavity wall 12 is constituted of a hard palate (front side) and the soft palate 16 (back side). The soft palate 16 is a soft mucous membrane portion at a rearward of the hard palate. The soft palate 16 includes a palatine velum and a uvula. The palatine velum cuts off a nasal cavity and an oral cavity during swallowing. The uvula is a portion hung down from the palatine velum. The oral cavity model 11 is constituted including each of the oral cavity organs. The gullet 13 and the respiratory tract 14 only need to include an entrance portion. The oral cavity model 11 is preferred to be formed according to the actual movements of the oral cavity organs. However, a movable part of the oral cavity model 11 may be limited for simplifying and facilitating the analysis. The organ properties of each of the oral cavity organs include, its dimensions, whether it is an elastic body or a rigid body, and if it is an elastic body, the elastic modulus, and related properties. The movements of each of the oral cavity organs include a movement, a rotation, a periodic movement and the like. When a food product is liquid, a physical property of the food product includes a fluid volume, a degree of viscosity, a surface tension, and a specific gravity. When the food product is a semisolid (with plasticity but without fluidity), the physical property includes an amount, a degree of viscosity, and a specific gravity. When the food product is a solid, the physical property includes a shape, dimensions, an elastic modulus, tensile strength, a yield point, yield point stress, shear rate dependence of degree of viscosity, dynamic viscoelasticity, static viscoelasticity, compressive stress, breaking stress, breaking strain, hardness, adhesiveness, cohesiveness and the like. A behavior of a pseudo food product while being swallowed is typically referred to as a behavior of movement from an oral cavity to a gullet through a throat. However, the behavior also includes cases where the pseudo food product returns to the oral cavity without reaching the throat or the gullet and cases where the pseudo food product enters the respiratory tract or the nasal cavity.

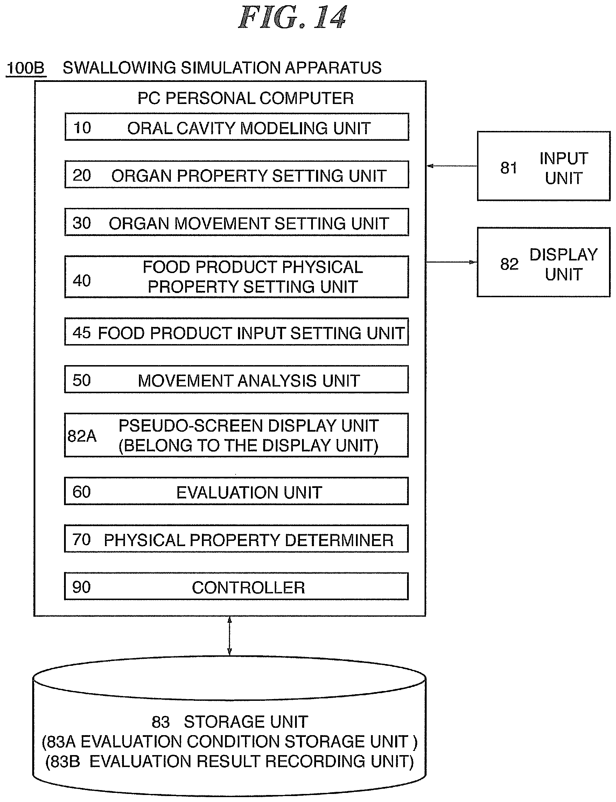

An input unit 81, for example, includes a computer mouse and/or a keyboard. Dragging the computer mouse pointer to an inside of the oral cavity of the oral cavity model 11 inputs the pseudo food product (includes a pseudo medicinal product or a pseudo quasi-drug). Or, a food product input setting unit 45 (see FIG. 14) may be disposed and the pseudo food product and an injection position and injection time of the pseudo food product may be preset so as to automatically inject the pseudo food product. A movement analysis unit 50 analyzes using a particle method. Moving particle-Semi-implicit (an MSP) method, for example, is applicable. As for "display on a moving screen" relating to a display unit 82, a display on the moving screen such as a liquid crystal display is typically used. Displaying the moving screen is useful for an evaluator to observe the moving screen for evaluation. However, automatic evaluation may be made (see second aspect). In the case where the evaluation is made by an evaluator, the evaluator observes the moving screen and inputs an evaluation result from the input unit 81 to an evaluation table displayed on the display unit 82.

With the configuration according to this aspect, the food product development assistance apparatus 200A sets the organ properties, the movements of the oral cavity organs, and the physical property of the food product in the oral cavity model 11. Then, the behavior of the food product is analyzed using the particle method. This facilitates reproduction of actual phenomenon of swallowing. Then, the swallowing phenomenon is analyzed using the swallowing simulation method, which facilitates the reproduction of actual phenomenon of swallowing, thus an easy-to-eat or easy-to-drink food product can be developed.

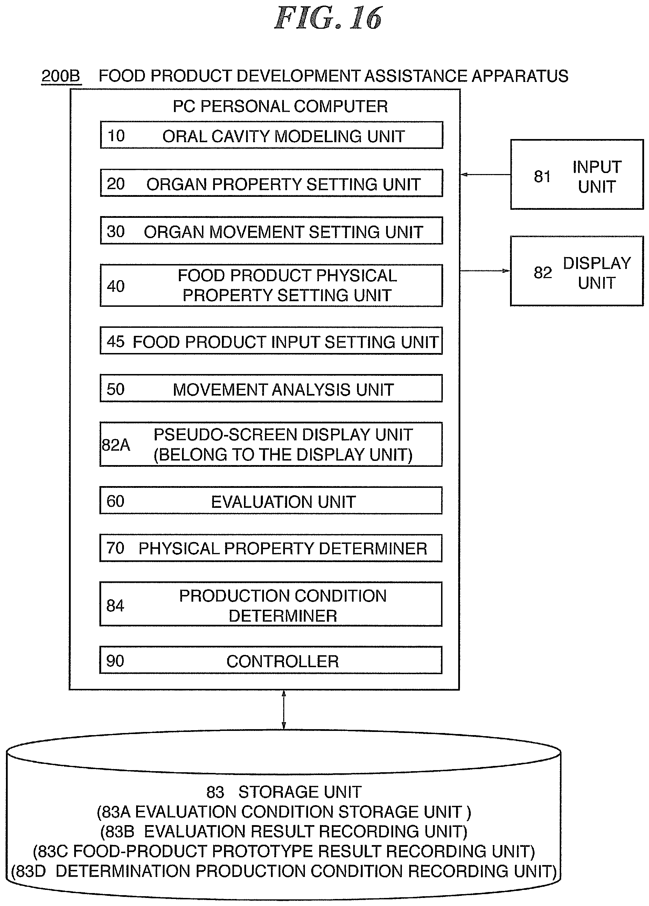

The food product development assistance apparatus 200B of the second aspect of the present invention is that according to the first aspect, as shown in, for example, FIG. 16, the apparatus comprises; an evaluation unit 60 configured to evaluate easiness of eating and/or easiness of drinking of the food product or similar product based on the behavior of the pseudo food product 41 while being swallowed on the moving screen; wherein, the moving screen is a virtual moving screen formed at a virtual space to simulatively display an analysis result of a movement of each of the oral cavity organs and a behavior of the pseudo food product 41 to 44 while being swallowed, the analysis result being analyzed by the movement analysis unit 50; and the evaluation unit 60 evaluates whether the behavior of the pseudo food product on the virtual moving screen meets a predetermined condition or not.

Here, the virtual moving screen is referred to as a virtual moving screen formed at a virtual space in a personal computer PC. However, the virtual moving screen displays the same contents as contents of the moving screen when displayed on the display unit 82. For automatic evaluation, the pseudo screen display unit 82A is disposed in the computer and the evaluation condition storage unit 83A is disposed in the storage unit 83. Analysis results are dynamically displayed on the virtual moving screen of the pseudo screen display unit 82A. Then, the analysis results are collated with the evaluation condition stored in the evaluation condition storage unit 83A for evaluation. An evaluation result is automatically stored in the evaluation result recording unit 83B of the storage unit 83. The pseudo screen display unit 82A belongs to the display unit 82. The virtual moving screen belongs to a moving screen of the display unit 82. The "display on a moving screen" also includes the case where the analysis results are thus dynamically displayed on the virtual moving screen. Predetermined conditions include, for example, the following. A food product does not enter the respiratory tract, not get stuck to the gullet, not adhere to the tongue or the gullet, a period from introduction in the oral cavity to having passed through the gullet is within a predetermined range, stress applied to a wall surface is equal to or less than a predetermined value, shear stress at the wall surface is equal to or less than a predetermined value or the like.

With the configuration according to this aspect, the analysis result displayed on the virtual moving screen and the evaluation condition stored in the evaluation condition storage unit 83A are collated. Thus, easiness of eating and/or easiness of drinking of the food product or similar product can be automatically evaluated.

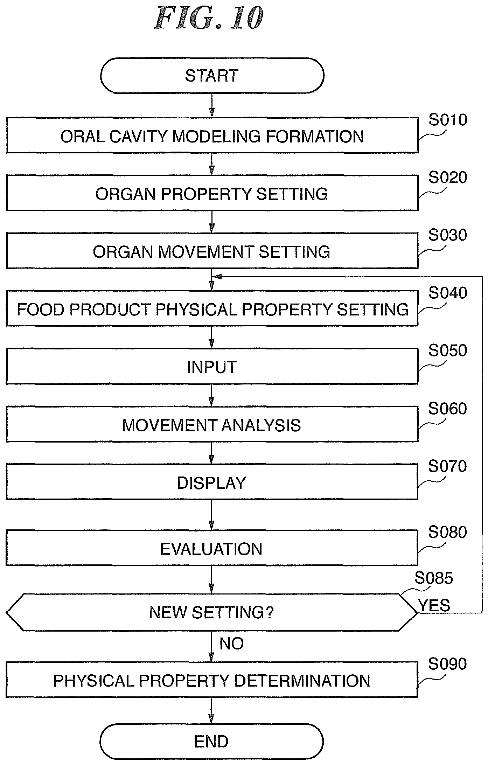

To solve the above described problems, a swallowing simulation method of the third aspect of the present invention comprises, as shown in, for example, FIG.10; an oral cavity modeling step (S010) of forming an oral cavity model 11 formed of oral cavity organs; an organ property setting step (S020) of setting an organ property of each of the oral cavity organs in the oral cavity model 11; an organ movement setting step (S030) of setting a movement of each of the oral cavity organs in the oral cavity model 11; a food product physical property setting step (S040) of setting a food product or similar product as an analysis target and a physical property of the food product or similar product; an input step (S050) of inputting a pseudo food product 41 to the oral cavity, the pseudo food product being formed by modeling the food product or similar product; a movement analysis step (S060) of
analyzing a movement of each of the oral cavity organs and a behavior of the pseudo food product 41 while being swallowed in the oral cavity model 11 using a particle method; a display step (S070) of displaying an analysis result of the movement of each of the oral cavity organs and the behavior of the pseudo food product 41 while being swallowed on a moving screen, the analysis result being analyzed in the movement analysis step (S060); an evaluation step (S080) of evaluating easiness of eating or easiness of drinking of the food product based on the analysis result of the behavior of the pseudo food product 41 while being swallowed; and a physical property determination step (S090) of determining the physical property of the food product or similar product regarded as appropriate in the evaluation step (S080).
A food product development method comprises a swallowing simulation method, and may further comprise a food product prototype production step (S110) of performing an experimental production under an appropriately set production condition to have the physical property determined as appropriate in the physical property determination step (S090); and a production condition determination step (S120) of determining a production condition that sets a physical property of the food product or similar product to the physical property determined as appropriate in the physical property determination step (S090) based on the result in the food product prototype production step (S110).

This aspect is a food product development method corresponding to the food product development assistance apparatus 200A. Here, the evaluation step (S080) includes the evaluation by a human and/or an automatic evaluation by the apparatus. In the physical property determination step (S090), for example, simulation with a plurality of physical property values determines an appropriate physical property range. The appropriate physical property may be one point of physical property. The plurality of physical properties allows creating a map within an appropriate range. Alternatively, the appropriate ranges may be classified into a plurality of levels (for example, rank A to rank C). Further, the food product prototype production step (S110) performs the experimental production under the appropriately set production condition that allows the prototype to have the physical property determined as appropriate in the physical property determination step (S090). The production condition determination step (S120) determines a production condition that sets the physical property of the food product to the physical property determined as appropriate in the physical property determination step (S090) based on the result in the food product prototype production step (S110). The production condition may be one point of condition or may specify a range. Alternatively, the production condition may determine an optimum value or may classify an appropriate range into ranks.

With the configuration according to this aspect, the swallowing phenomenon is analyzed using the swallowing simulation method, which facilitates reproduction of actual phenomenon of swallowing. This allows developing an easy-to-eat or easy-to-drink food product.

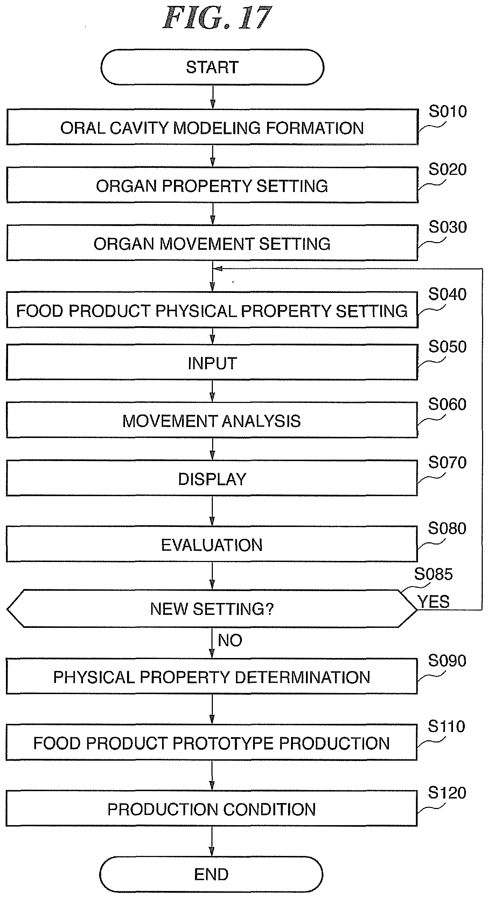

The food product development method of the fourth aspect of the present invention is that according to the third aspect, as shown in, for example, FIG. 17; wherein the moving screen is a virtual moving screen formed at a virtual space by a food product development assistance apparatus 200B to simulatively display an analysis result of a movement of each of the oral cavity organs and a behavior of the pseudo food product 41 while being swallowed, the analysis result being analyzed in the movement analysis step (S060); the display step is a virtual display step (S075) to simulatively display the analysis result on the virtual moving screen; and the evaluation step (S080) evaluates whether the behavior of the pseudo food product on the virtual moving screen simulatively displayed in the virtual display step (S075) meets a predetermined condition or not.

With the configuration according to this aspect, the food product development assistance apparatus 200B can collate the analysis result displayed on the virtual moving screen and the evaluation condition stored in the evaluation condition storage unit 83A. Thus, easiness of eating or easiness of drinking of the food product or similar product can be automatically evaluated.

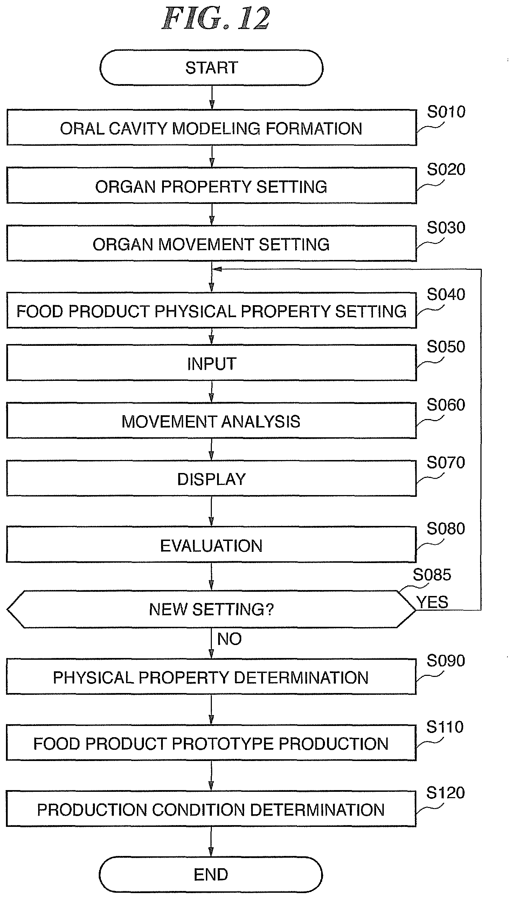

The food product development method of the fifth aspect of the present invention is that according to the third aspect or the forth aspect, as shown in, for example, FIG. 12; wherein the method repeatedly performs from the food product physical property setting step (S040) to the evaluation step (S080) while the physical property of the food product or similar product is changed and set in the food product physical property setting step (S040).

With the configuration according to this aspect, a plurality of simulation about food product or similar product with different physical properties are performed and are compared. Thus, an appropriate physical property range can be obtained. Additionally, an optimum physical property can be selected among simulated physical properties. For handling the plurality of physical properties, an appropriate physical property map can be created in a multidimensional space.

The food product development method of the sixth aspect of the present invention is that according to any one of the third aspect to the fifth aspect, as shown in, for example, FIG. 3; wherein the organ property setting step (S020) sets an oral cavity wall 12 as a rigid body and a tongue 15 as an elastic body; the organ movement setting step (S030) sets a plurality of moving walls 18 in the tongue 15, the tongue 15 being set so as to move in a peristaltic movement or a wave movement by moving the plurality of moving walls 18 to a direction intersecting with a surface of the tongue 15 with a predetermined period and a predetermined phase difference, and sets a soft palate 16 , an epiglottis 17, and a gullet wall 19 so as to move together with a predetermined phase difference to the peristaltic movement or the wave movement; and the movement analysis step (S060) treats the tongue 15 and the pseudo food product 41 as particles.

Here, the surface of the tongue 15 is referred to as a surface of a near side (upper side). The peristaltic movement is referred to as a simulated movement of a movement of a digestive system such as a large bowel, a small bowel or the like. Meanwhile, a wave movement is referred to as a simulated movement of a movement of wave.

With the configuration according to this aspect, setting movements of a plurality of moving walls 18 to the same period and a shifting phase allows a peristaltic movement or the wave movement of the tongue to be reproduced close to the actual phenomenon. This allows approximate reproduction of the actual phenomenon in the swallowing phenomenon.

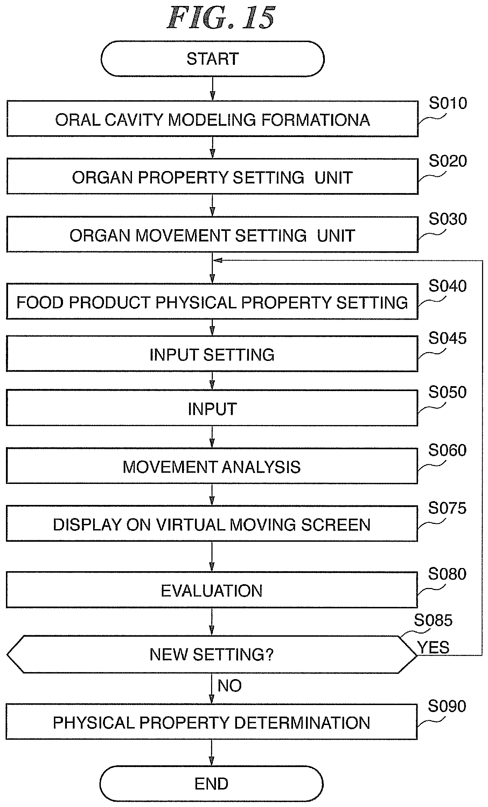

The food product development method of the seventh aspect of the present invention is that according to any one of the third aspect to the sixth aspect, as shown in, for example, FIG. 15; wherein the organ movement setting step (S030) sets a movement of a soft palate 16 and an epiglottis 17 as a movement of a rotator where a rotational center moves.

With the configuration according to this aspect, movements of the soft palate 16 and the epiglottis 17 can be reproduced close to the actual phenomena.

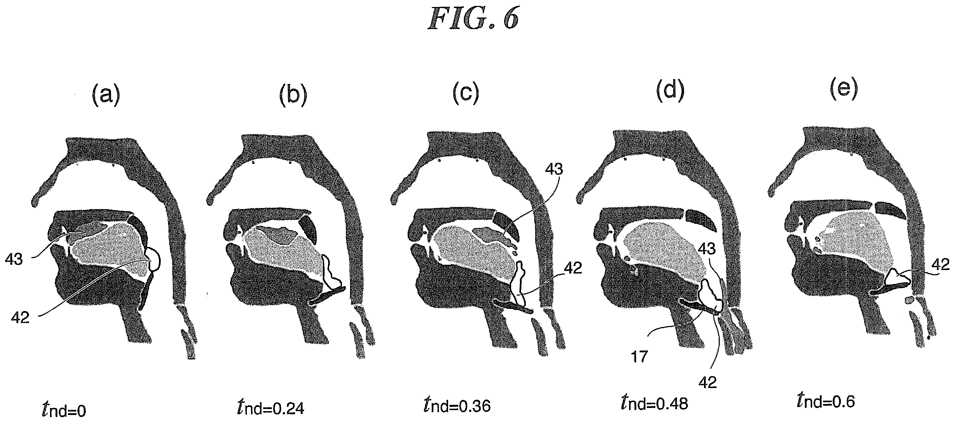

The food product development method of the eighth aspect of the present invention is that according to any one of the third aspect to the seventh aspect, as shown in, for example, FIG. 6; wherein the food product physical property setting step (S040) sets a plurality of liquid, semisolid, or solid pseudo food products 42, 43 with different physical property as an analysis target; and the movement analysis step (S060) determines free surfaces of a plurality of the pseudo food products 42, 43 and boundaries between the plurality of pseudo food products 42, 43, the movement analysis step (S060) analyzing a gearing behavior of the plurality of pseudo food products 42, 43.

With the configuration according to this aspect, the gearing behavior of the plurality of pseudo food products can be reproduced close to the actual phenomenon, and it is effective in analysis of the gearing behavior.

The food product development method of the ninth aspect of the present invention is that according to any one of the third aspect to the eighth aspect, as shown in, for example, FIG. 12; wherein the oral cavity modeling step (S010) forms a two dimensional oral cavity model 11; and the movement analysis step (S060) analyzes the behavior of the pseudo food product 41 in a two dimensional space.

With the configuration according to this aspect, the behavior of the pseudo food product 41 in the oral cavity model 11 is approximately expressed in a two dimensional space. This allows efficiently evaluating easiness of eating or easiness of drinking of the food product based on a simple analysis.

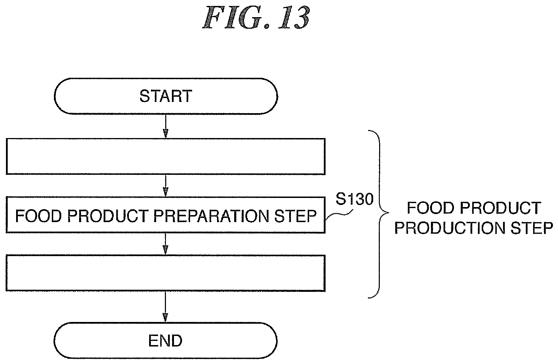

The food product development method of the tenth aspect of the present invention comprises, as shown in, for example, FIG. 13; a food product preparation step (S130) of preparing the food product using a production condition determined as a production condition for the physical property determined as appropriate in the physical property determination step (S090), in the production condition determination step (S120) of the food product development method according to any one of the third aspect to the ninth aspect.

Here, typically, the food product development method repeatedly performs simulation to check for an appropriate physical property. Then, the food product is developed by determining the production condition such that the physical property of the food product becomes appropriate. In a food product production method, when a food product is produced using the production condition determined in the production condition determination step of the food product development method in any of step in the production process (for example, a raw material combination step or a baking step), this corresponds to the aspect.

With the configuration according to this aspect, the food product development method allows reliably producing a food product excellent in easiness of eating or easiness of drinking.

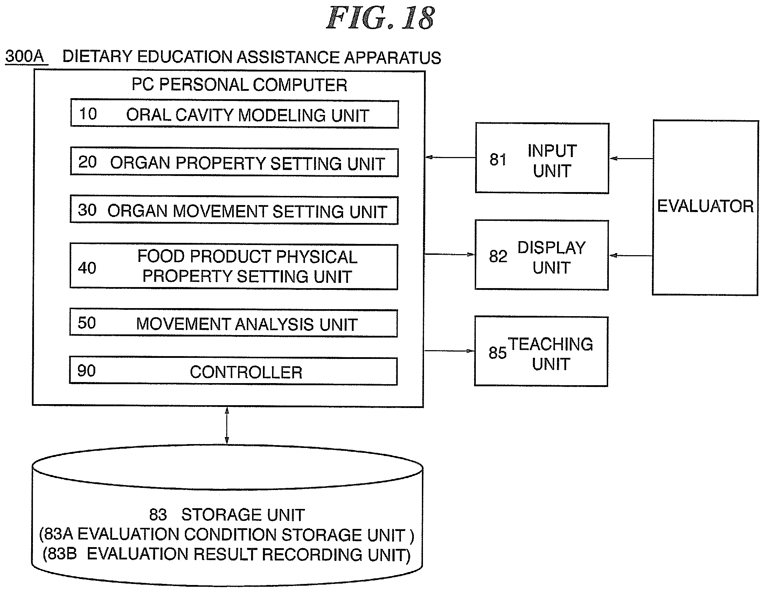

To solve the above described problems, the dietary education assistance apparatus 300A according to the eleventh aspect of the present invention comprises, as shown in, for example, FIG. 18; an oral cavity modeling unit 10 configured to form an oral cavity model formed of oral cavity organs; an organ property setting unit 20 configured to set an organ property of each of the oral cavity organs in the oral cavity model 11; an organ movement setting unit 30 configured to set a movement of each of the oral cavity organs in the oral cavity model 11; a food product physical property setting unit 40 configured to set a food product or similar product as an analysis target and a physical property of the food product or similar product; an input unit 81 configured to input a pseudo food product 41 to the oral cavity, the pseudo food product being formed by modeling the food product or similar product; a movement analysis unit 50 configured to analyze a movement of each of the oral cavity organs and a behavior of the pseudo food product 41 while being swallowed in the oral cavity model using a particle method; a display unit 82 configured to display an analysis result of the movement of each of the oral cavity organs and the behavior of the pseudo food product 41 while being swallowed on a moving screen, the analysis result being analyzed by the movement analysis unit 50; an evaluation result recording unit 83B configured to record an evaluation result of easiness of eating or easiness of drinking of the food product or similar product based on the analysis result of the behavior of the pseudo food product 41 while being swallowed; and a teaching unit 85 configured to teach the behavior of the pseudo food product 41 while being swallowed displayed on the moving screen by the display unit 82 associating with the evaluation result of the food product recorded in the evaluation result recording unit 83B.

This aspect is the dietary education assistance apparatus 300A to which is applied the food product development assistance apparatus 200A according to the first aspect.

With the configuration according to this aspect, the swallowing phenomenon is displayed using the swallowing simulation method, which facilitates reproduction of the actual phenomenon of a swallowing. Accordingly, easiness of eating or easiness of drinking of a food product is easily understood, and the apparatus is effective in dietary education.

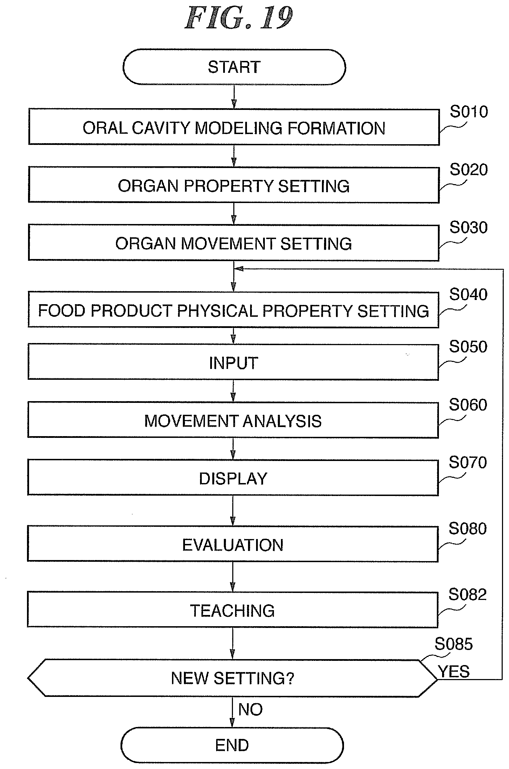

To solve the above described problems, the dietary education method according to a twelfth aspect of the present invention comprises, as shown in, for example, FIG. 19; an oral cavity modeling step (S010) of forming an oral cavity model 11 formed of oral cavity organs; an organ property setting step (S020) of setting an organ property of each of the oral cavity organs in the oral cavity model 11; an organ movement setting step (S030) of setting a movement of each of the oral cavity organs in the oral cavity model 11; a food product physical property setting step (S040) of setting a food product or similar product as an analysis target and a physical property of the food product or similar product; an input step (S050) of inputting a pseudo food product to the oral cavity, the pseudo food product being formed by modeling the food product or similar product; a movement analysis step (S060) of analyzing a movement of each of the oral cavity organs and a behavior of the pseudo food product 41 while being swallowed in the oral cavity model 11 using a particle method; a display step (S070) of displaying an analysis result of the movement of each of the oral cavity organs and the behavior of the pseudo food product 41 while being swallowed on a moving screen, the analysis result being analyzed in the movement analysis step (S060); an evaluation step (S080) of evaluating easiness of eating or easiness of drinking of the food product or similar product based on the analysis result of the behavior of the pseudo food product 41 while being swallowed; and a teaching step (S082) of teaching the behavior of the pseudo food product 41 while being swallowed displayed on the moving screen in the display step (S070) associating with the evaluation result of the food product evaluated in the evaluation step (S080).

This aspect is a dietary education method corresponding to the dietary education assistance apparatus 300A according to the eleventh aspect.

With the configuration according to this aspect, the swallowing phenomenon is displayed using the swallowing simulation method, which facilitates reproduction of the actual phenomenon of swallowing. Accordingly, easiness of eating or easiness of drinking of a food product is easily understood, and the method is effective in dietary education.

### Effect of the Invention

According to the present invention, a swallowing phenomenon is analyzed using a swallowing simulation method, which facilitates reproduction of an actual phenomenon of swallowing. This allows developing an easy-to-eat or easy-to-drink food product.

### Brief description of drawings

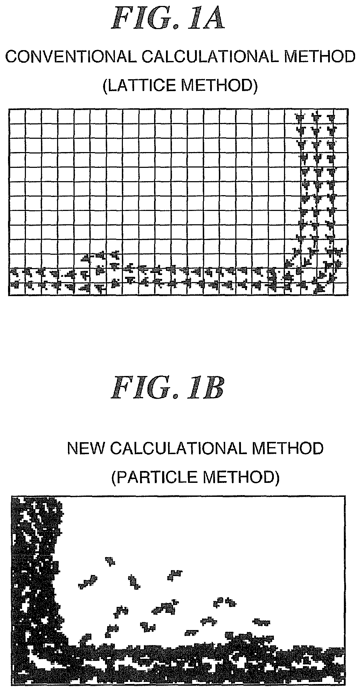

FIG. 1A describes a lattice method (conventional analysis method) .
FIG. 1B describes a particle method (new analysis method)

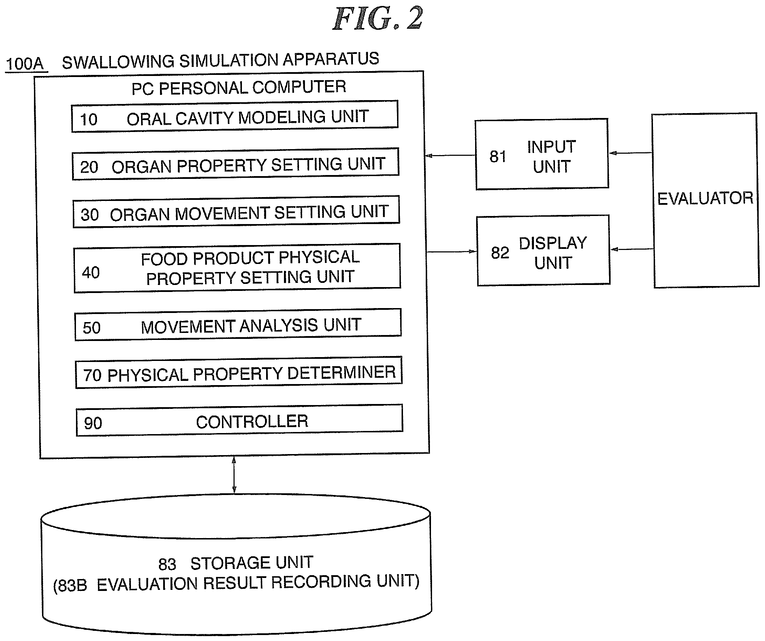

FIG. 2 illustrates an exemplary configuration of a swallowing simulation apparatus according to a first embodiment.
FIG. 3 illustrates an exemplary oral cavity model.

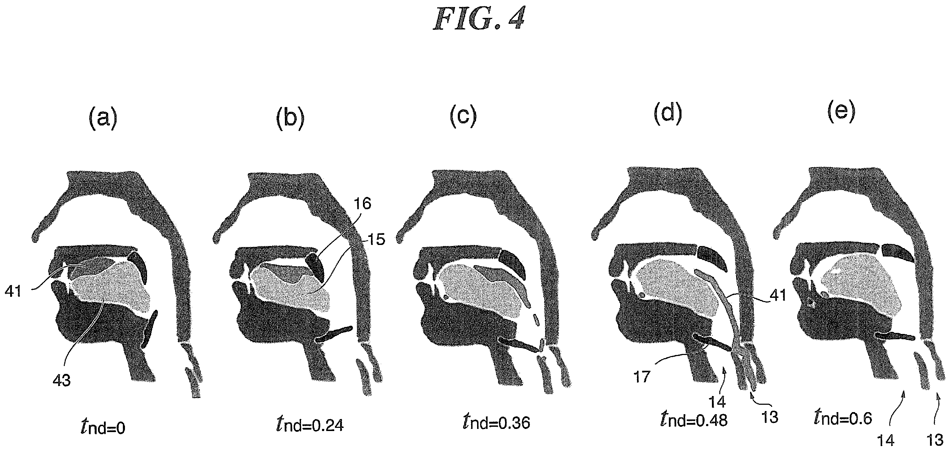

FIG. 4 simulatively illustrates a swallowing phenomenon of water.

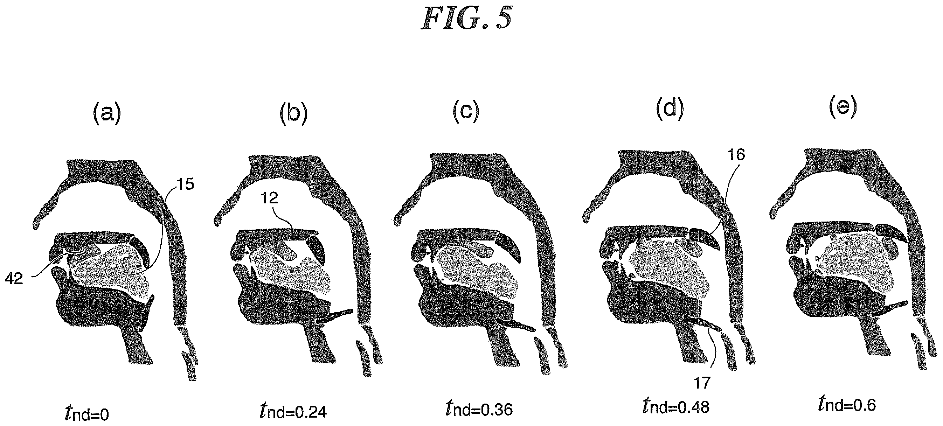

FIG. 5 simulatively illustrates the swallowing phenomenon of an adherent=bolus (assume a rice cake).
FIG. 6 simulatively illustrates the swallowing phenomenon when the adherent bolus obstructing near the soft palate is rinsed with water.

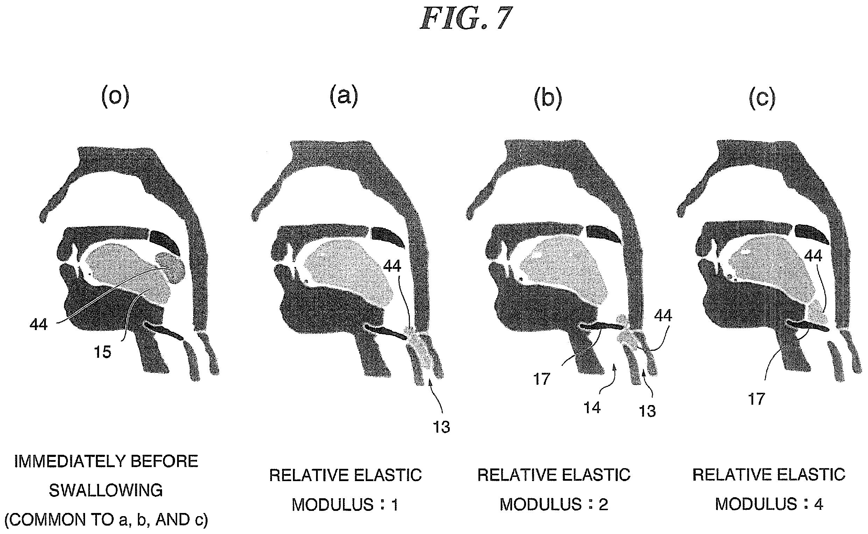

FIG. 7 illustrates swallowing simulation results of a jelly-like bolus.

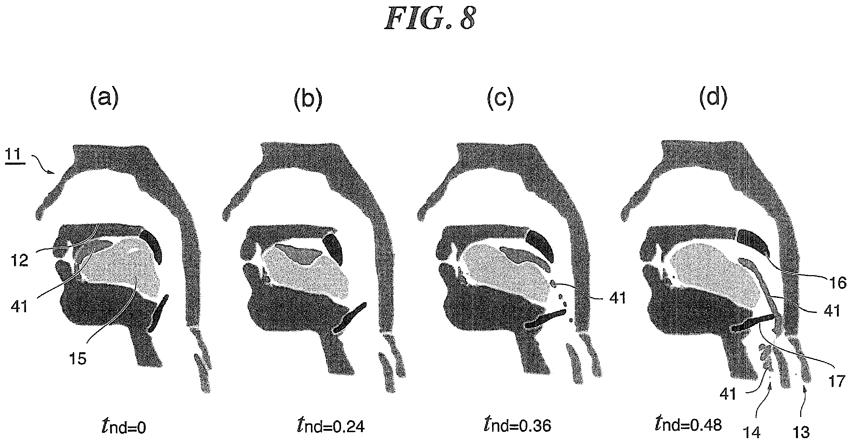

FIG. 8 simulatively illustrates the swallowing phenomenon in the case where a movement of an epiglottis is slow.

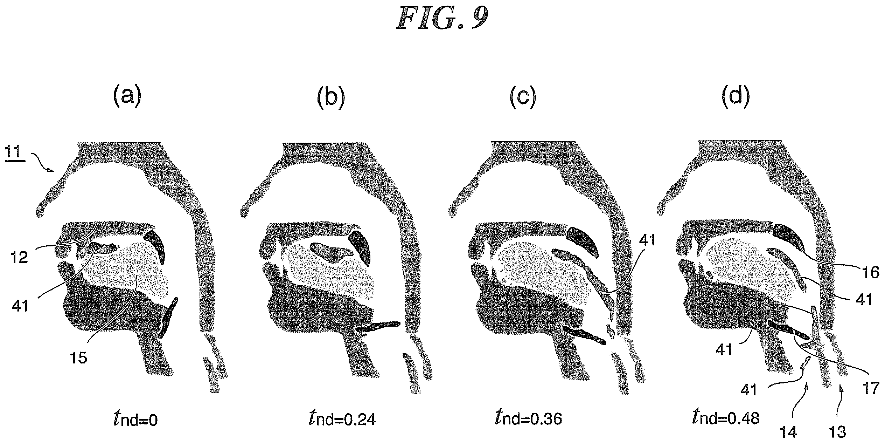

FIG. 9 simulatively illustrates the swallowing phenomenon in the case where a movement of a boundary surface between a gullet and a respiratory tract is slow.
FIG. 10 illustrates an exemplary processing flow of the swallowing simulation method according to the first embodiment.
FIG. 11 illustrates an exemplary configuration of a food product development assistance apparatus according to a first embodiment.
FIG. 12 illustrates an exemplary processing flow of the food product development method according to a first embodiment.
FIG. 13 illustrates an exemplary processing flow of the food product production method according to a first embodiment.
FIG. 14 illustrates an exemplary configuration of the swallowing simulation apparatus according to a second embodiment.
FIG. 15 illustrates an exemplary processing flow of the swallowing simulation method according to the second embodiment.
FIG. 16 illustrates an exemplary configuration of a food product development assistance apparatus according to a second embodiment.
FIG. 17 illustrates an exemplary processing flow of a food product development method according to a second embodiment.
FIG. 18 illustrates an exemplary configuration of a dietary education assistance apparatus according to a fifth embodiment.
FIG. 19 illustrates an exemplary processing flow of dietary education method according to a fifth embodiment.

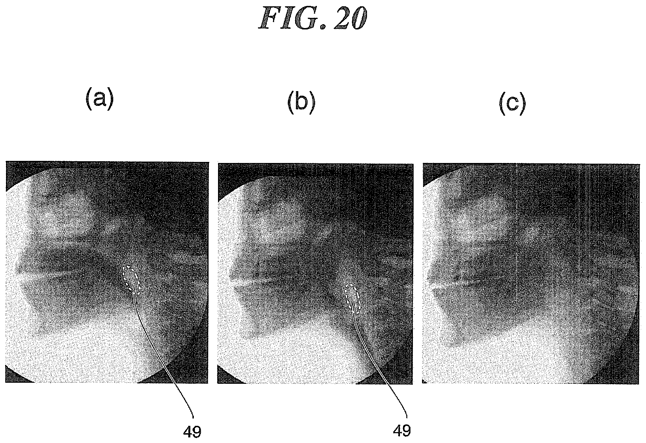

FIG. 20 illustrates an exemplary videofluoroscopic swallowing.

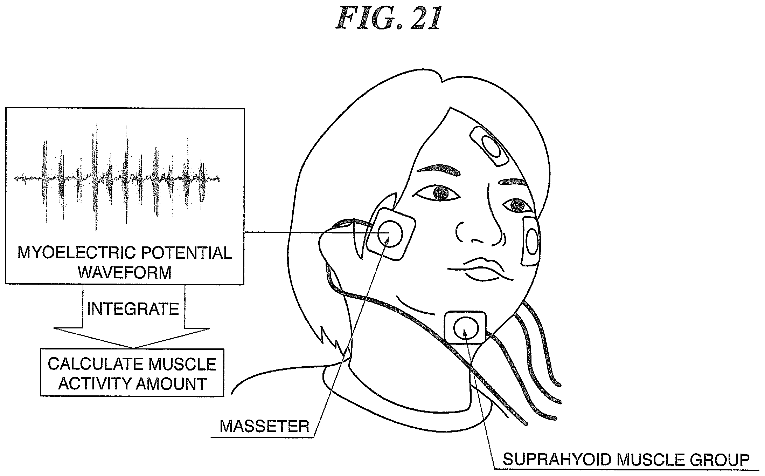

FIG. 21 illustrates an exemplary myoelectric potential measurement.

### Best mode for carrying out the invention

The present application is based on Japanese Patent Application No. 2011-146781 filed on June 30, 2011 in Japan. The present invention will be more completely understood by the detailed description provided hereinafter. Further areas of applicability of the invention will become more apparent from the detailed description provided hereinafter. However, it should be understood that the detailed description and specific examples indicate desired embodiments of the invention, and are provided for the purpose of illustration only.

Embodiments of the present invention will be described hereinafter in detail with reference to the drawings. In each drawing, like numerals and symbols will be used for identical or like elements, and duplicate descriptions may not be repeated.

### (Particle Method)

According to the embodiment, as an analysis method that allows expressing a large deformation of a liquid surface, a spray and the like, the particle method that treats liquid and solid analysis targets as particles is employed for simulations. First, the particle method will be described.

FIG. 1A and FIG 1B illustrate a difference between a lattice method, which is the conventional analysis method, and the particle method, which is a new analysis method. FIG. 1A illustrates a conceptual diagram of the lattice method while FIG. 1B illustrates a conceptual diagram of the particle method. The lattice method divides an analysis region by grid and calculates physical quantities of each grid. That is, a change in the liquid surface goes along a shape of the grid. Accordingly, an analysis of a case when the spray occurs or the liquid surface is largely deformed is difficult. In contrast to this, the particle method, especially an Moving particle-Semi-implicit (MPS) method is comparatively new analysis method, which was developed in 1995 (Koshizuka et al, Comput.Fluid Dynamics J, 4, 29-46, 1995). The particle method replaces a fluid with particles and calculates the physical quantities of each particle. As a result, a subtle change in the liquid surface can be analyzed, allowing an analysis when the spray occurs or the liquid surface is largely deformed. However, fluids or boluses in vivo have not been analyzed using the particle method up to the present. Therefore, the inventors have developed the simulation apparatus and the simulation method where the particle method is applied to estimation of behavior of the liquid or the bolus in the living body. The following describes the embodiments.

In the MPS method, as a governing equation for an incompressible flow, a conservation-of-mass formula and a conservation-of-momentum formula are established. Lagrangian derivative may be used for the time derivative in the conservation-of-momentum formula. Terms expressing movement and flow need not be denoted explicitly. A weighting function w(r) (a function of a distance r between particles, and expressed by w(r) = r_{E}/r - 1; 0 ≤ r < r_{E}, and w(r) = 0; r_{E} < r, being a decreasing function of the distance r between particles within a constant separation r_{E}, being 0 with outside the constant distance r_{E}) is introduced, and the weighting function is used for particle interaction. A Laplacian model is established on the physical quantities in the positions of each particle in the particle interaction model, and the discretization equations are solved. Solving this discretization equation in accordance with a solution method of a matrix equation, a speed is obtained. Then the position of each particle is determined.

A simulator (analysis software) to perform the swallowing simulation method according to the embodiment models the oral cavity organs and analyzes the behaviors of the fluid or the bolus while passing through the oral cavity and the throat using the particle method.

From the analysis results using the simulator, for example, the following are performed.
(a) Estimations of the swallowing and risks of an accidental swallowing and an accidental ingestion depending on the difference in a physical property value of a food product or similar product
(b) Estimations of a swallowing period depending on the difference in the physical property value of the food product or similar product
(c) Estimations of a force and shear stress applied to the throat wall depending on the difference in the physical property value of the food product or similar product
(d) Evaluations on easiness of drinking, easiness of eating, difficulty of drinking, and difficulty of eating based on the correlations between the above described data and a sensory evaluation.

The evaluations are made by the evaluator or automatically made by the swallowing simulation apparatus.

### (First embodiment)

### (Swallowing Simulation Apparatus Configuration)

FIG. 2 illustrates an exemplary configuration of the swallowing simulation apparatus 100A according to the first embodiment. The first embodiment describes an exemplary swallowing evaluation made by the evaluator's inputting the food product and viewing the moving image.

The swallowing simulation apparatus 100A includes an oral cavity modeling unit 10, an organ property setting unit 20, an organ movement setting unit 30, a food product physical property setting unit 40, an input unit 81, a movement analysis unit 50, a display unit 82, a physical property determiner 70, a controller 90, and a storage unit 83. The oral cavity modeling unit 10 forms an oral cavity model formed of oral cavity organs. The organ property setting unit 20 sets an organ property of each of the oral cavity organs in the oral cavity model. The organ movement setting unit 30 sets a movement of each of the oral cavity organs in the oral cavity model. The food product physical property setting unit 40 sets a food product as an analysis target and a physical property of the food product. The input unit 81 inputs a pseudo food product, which is formed by modeling the food product, to the oral cavity. The movement analysis unit 50 analyzes a movement of each of the oral cavity organs and a behavior of the pseudo food product while being swallowed in the oral cavity model using a particle method. The display unit 82 displays analysis results of the movement of each of the oral cavity organs and the behavior of the pseudo food product while being swallowed analyzed by the movement analysis unit 50 on a moving screen. The physical property determiner 70 determines a physical property of a food product or similar product regarded as appropriate based on the evaluation result. The controller 90 controls the swallowing simulation apparatus 100A and each unit of the swallowing simulation apparatus 100A to have functions required for the swallowing simulation apparatus 100A. The storage unit 83 stores the oral cavity model, the organ properties, the setting conditions, the analysis results, and the evaluation results. Among these units, the oral cavity modeling unit 10, the organ property setting unit 20, the organ movement setting unit 30, the food product physical property setting unit 40, the movement analysis unit 50, the physical property determiner 70, and the controller 90 can be realized in the personal computer PC and disposed inside of the personal computer PC. The evaluator makes evaluations observing the moving screen on the display unit 82 and inputs the evaluation results from the input unit 81. The input evaluation results are recorded in the evaluation result recording unit 83B of the storage unit 83. In the present invention, an aspect where the physical property is determined by the human (for example, the evaluator) is also possible (see a fourth embodiment).

FIG. 3 illustrates the exemplary oral cavity model 11. FIG. 3(a) illustrates a movable portion in the model. FIG. 3(b) illustrates a moving wall 18 (performs a peristaltic movement) portion of the tongue 15 in the model. In the embodiment, an exemplary peristaltic movement by the four moving walls 18 is illustrated. The oral cavity modeling unit 10 forms the oral cavity model 11 formed of oral cavity organs including, the oral cavity wall 12, the gullet 13 (entrance portion is illustrated), the respiratory tract 14 (entrance portion is illustrated), the tongue 15, the soft palate 16, and the epiglottis 17 and the like. The organ properties of each of the oral cavity organs (classification of rigid body, elastic body, plastic body, viscous body, powder, fluid or the like and physical property such as elastic modulus and degree of viscosity are set by the organ property setting unit 20. For simplification, the tongue 15, the soft palate 16, the epiglottis 17, and the gullet 13 entrance are set as an elastic body while the others are set as a rigid body. The movements of the oral cavity organs (such as a reciprocation, a rotational movement, a peristaltic movement and the like) are set by the organ movement setting unit 30. For simplification, the movement of the tongue 15 is expressed by the peristaltic movement, those of the soft palate 16 and the epiglottis 17 is expressed by reciprocation at the base and rotational movement around the base, and the entrance portion of the gullet 13 is expressed by the reciprocations in the perpendicular direction to the central axis of the gullet 13. The wave movement can be used instead of the peristaltic movement.

Now returning to FIG. 2, as the target for the swallowing simulation, a medicinal product, a quasi-drug can be used as well as a food product ("the food product, the medicinal product, or the quasi-drug" is referred to as a "food product or similar product"). When the food product or similar product is liquid, the food product physical property setting unit 40 sets physical properties such as a fluid volume, a degree of viscosity, a surface tension, a specific gravity and the like. When the food product or similar product is a solid, the food product physical property setting unit 40 sets physical properties such as a shape, dimensions, an elastic modulus, tensile=strength, a yield point, yield point stress, shear rate dependence of degree of viscosity, dynamic viscoelasticity, static viscoelasticity, compressive stress, breaking stress, breaking strain, hardness, adhesiveness, cohesiveness and the like. When the food product or similar product is a semisolid (with plasticity but without fluidity), the food product physical property setting unit 40 sets physical properties such as an amount, a degree of viscosity, a specific gravity, a yield point, yield point stress, shear rate dependence of degree of viscosity, dynamic viscoelasticity, static viscoelasticity, compressive stress, adhesiveness, cohesiveness and the like.

The input unit 81 is configured of an input device such as the computer mouse, the keyboard and the like. The input unit 81 injects a pseudo food product to be injected in the oral cavity. The computer mouse pointer, for example, is dragged in the oral cavity, an injection position of the pseudo food product in the oral cavity is, for example, set near the teeth in the oral cavity (for example, within 1/2 length of the pseudo food product), and time immediately after the dragging is set as injection time.

The movement analysis unit 50 analyzes a behavior of the pseudo food product while being swallowed in association with movements of the oral cavity organs. The movement of the tongue 15 is expressed by a peristaltic movement or a wave movement, and the movements of soft palate 16 and the epiglottis 17 are expressed by reciprocation at the base and rotational movement around the base. The reciprocation of the gullet 13 entrance moves the food product or similar product injected in the oral cavity. The movement of the food product or similar product is analyzed using the particle method. The food product or similar product is treated as particles in any forms of solid, semisolid, and liquid.

The display unit 82 displays an analysis result of the behavior of the food product or similar product on the moving screen. One exposure of the moving image can be displayed as a still image. Tracing back the time and displaying the moving images while being rewound are also possible. The storage unit 83 stores, an oral cavity model, organ properties, a setting condition, an analysis result, an evaluation result and the like.

The evaluation is made by the evaluator viewing the moving screen on the display unit 82. "Good", "poor", a rank, a score, or similar evaluation is input to a cell in an evaluation table displayed on the display unit 82, for example. The evaluation result is recorded in the evaluation result recording unit 83B. An appropriate physical property value of the food product or similar product can be obtained by making evaluation while changing the physical property value of the food product or similar product by the food product physical property setting unit 40. The physical property determiner 70 automatically determines the physical property of the food product or similar product regarded as appropriate based on the evaluation result recorded in the evaluation result recording unit 83B. The number of physical properties may be a single or plural. The appropriate physical property may be, for example, indicated by creating a map showing an appropriate range, may be indicated by classification into a plurality of levels (for example, rank A to rank C), may be indicated by plurality of points, or may be indicated by an optimum one point. When many physical properties are to be obtained, the appropriate physical property range may be obtained using multidimensional analysis of principal component.

The controller 90 controls the swallowing simulation apparatus 100A and each unit of the swallowing simulation apparatus 100A to have functions required for the swallowing simulation apparatus 100A. The controller 90 includes a swallowing simulator (analysis software) in a built-in memory.

### (Swallowing Simulator)

The swallowing simulator has been created using a general-purpose two-dimensional particle method analysis software "Physi-Cafe" (manufactured by Prometech Software, Inc.). A physical property value of a fluid and time, for example, cannot be directly input to the analysis software as a numerical value. However, dimensionless physical quantities of the physical property value of the fluid and time can be appropriately changed, featuring a high speed analysis by simplifying a qualitative analysis.

FIG. 3(a) illustrates a movable portion in the oral cavity model 11. FIG. 3(b) illustrates the moving wall 18 (performs a peristaltic movement) portion of the tongue 15 in the model. In the model, for simplification, only the four portions are set as the movable parts: the tongue 15, the soft palate 16, the epiglottis 17, and an entrance of the gullet 13. A mechanism of transporting a bolus rearward by the peristaltic movement is configured as follows. The four moving walls 18 are embedded in the tongue 15, which is an elastic body. Then, the movement is performed while changing an amplitude of oscillation at the same period and shifting a phase. In this model, the four moving walls 18 are set as the elastic body. The one moving wall 18 cannot achieve the peristaltic movement, the two moving walls 18 generates an awkward movement, the three or more moving walls 18 can express a smooth peristaltic movement. The five or more moving walls 18 increase a computational load whereas makes little difference in natural movement from the case where the three or four moving walls 18 are used. Accordingly, use of the three or four pieces is preferable. Thus, a simulation operation where the elastic body (tongue) autonomously deforms is achieved. Then, a forcible deformation of the elastic body, which is extremely difficult in a usual analysis, can be expressed. This respect is distinctive in numerical analysis (simulation). Additionally, as illustrated in FIG. 3(b), the pseudo food product and the tongue 15 are all constituted by particles, regardless of whether the pseudo food product or the tongue 15 is liquid or solid.

Table 1 illustrates movements of the movable parts. The main feature is that a movement amount of displacement and angle are provided by a function. In particular, use of a periodic function achieves consecutive simulations. A to D in Table 1 are moving walls and disposed in the order of A, B, C, and D from the left in FIG. 3(b).

**TABLE 1 THE RELATION BETWEEN MOVING PART AND MOVEMENT AMOUNT IN STANDARD MOTION**

| MOVING PART | | MOVEMENT AMOUNT | | |
|---|---|---|---|---|
| | | X DIRECTION | Y DIRECTION | ROTATION |
| TONGUE | A | -1*sin(t)-0.5 | 2*sin(t-1) | - |
| | B | -3*sin(t) | -3*sin(t)-1 | - |
| | C | -sin(t-1)-1.0 | -4*sin(t-1) | - |
| | D | -2.0*sin(t)-2.0 | -2*sin(t-3)+2 | - |
| SOFT PALATE | | -2*sin(t) | 0 | 0.5*sin(t-3) |
| EPIGLOTTIS | | sin(t+1.2)-0.8 | -sin(t+1.2)+1 | 0.8*sin(t+1.2)-0.6 |
| GULLET WALL | | 2*sin(t+1.2) | -2*sin(t+1.2) | 0 |

The movement amount of each organ can be easily changed by changing a formula in Table 1 and a parameter of the formula. Specifically, the movement amount can be adjusted by changing an amplitude of a sine function. A speed and timing of the movement can be adjusted by changing the period and the phase. The simulator features a high degree of freedom in adjustment of each portion.

### (Analysis Case 1)

FIG. 4 illustrates an exemplary analysis of a swallowing value experiment where water (assuming a degree of viscosity of 1 mPa·s) 41 is simulated. Here, a dimensionless swallowing period is denoted as t_{nd}. The t_{nd} is what an analysis period taken for one swallowing (25 sec) is divided by a period taken for an actual swallowing phenomenon to complete (defined that the swallowing action is completed after an elapse of 1 sec from entrance of the water 41 in the mouth in this analysis).

The liquid (water) 41 that exists on the tongue 15 at t_{nd} = 0 is held between the tongue 15 and the soft palate 16 at t_{nd} = 0.24. At t_{nd} = 0.36, it is seen that the soft palate 16 moves rearward and rotates-to form a space for the liquid 41 to pass through whereas the soft palate 16 obstructs the passage from the nasal cavity. At t_{nd}= 0.48, it is seen that the liquid 41 flows to the gullet 13 without entering the respiratory tract 14 lidded by the epiglottis 17. At t_{nd}= 0.6, it is seen that the water 41 does not exist around the epiglottis 17 when the epiglottis 17 rises, thus accidental swallowing and accidental ingestion do not occur. It can also be observed from this result that a complicated fluid behavior involving a free surface, which was difficult to be expressed by the analysis method (lattice method) up to the present, can be expressed by the particle method.

### (Analysis Case 2)

FIG. 5 illustrates the simulation results of a bolus such as a rice cake 42 with high adhesiveness while being swallowed. The analysis software, which is the base of the simulator that has been developed this time, treats a physical property value, such as adhesiveness, as a relative value with a physical property value of a certain standard object, not an absolute value. Therefore, in the simulation, adhesiveness was appropriately changed (about 600 to 2300 J/m³) for analysis to the extent of adhering to a palate. The adherent bolus 42 that exists on the tongue 15 at t_{nd} = 0 adheres to the oral cavity wall 12 (hard palate) at t_{nd} = 0.24, and a rearward flow is not observed. At t_{nd}= 0.36, it is seen that the bolus 42 is stretched while adhering to the palate in spite of the peristaltic movement of the tongue 15. At t_{ad} = 0.48, it is seen that the bolus 42 adheres to the soft palate 16 and does not come out although being lidded by the epiglottis 17. Finally, even at t_{nd}= 0.6, the adherent bolus 42 firmly adheres to the soft palate 16.

FIG. 6 illustrates a simulation result of a state where the adherent bolus 42 obstructs near the soft palate 16, rinse liquid (assuming water) 43 is run into the mouth, and the obstructing bolus 42 is washed away. At t_{nd} = 0.36, the rinse water 43 being run into the mouth flows to the larynx. However, even at t_{nd}= 0.48, the adherent bolus 42 remains at the epiglottis 17. Thus, it can be observed that washing away the adherent bolus 42 by one rinsing is difficult. This simulator also confirmed that, similarly to the actual phenomenon, the bolus with high adhesiveness needs to be rinsed by plural times.

Thus, the simulator can couple the two or more liquid, solid, and/or semisolid of boluses and/or fluids with different degree of viscosity, adhesiveness, a surface tension, or similar physical property for solution. Coupled analyses of liquid-liquid, liquid-solid, and solid-solid with free surface and different physical property have been extremely difficult up to the present. However, use of the particle method facilitates qualitative analysis.

### (Analysis Case 3)

FIG. 7 illustrates a simulation result when the bolus 44 that can be broken under a certain amount of constant force, such as a jelly, is being swallowed. Here, hardness of the bolus 44 is expressed using a relative elastic modulus, which is a relative ratio with a standard bolus. Shapes of the boluses 44 immediately before being swallowed are all same.

FIG. 7(o) illustrates a state immediately before the swallowing. FIG. 7(a) illustrates a case where the relative elastic modulus of the bolus 44 is low (relative elastic modulus = 1). It can be seen that the bolus 44 deforms along the shape of the gullet 13 at the moment of entrance to the gullet 13 and then flows. FIG. 7(b) illustrates a case where the relative elastic modulus of the bolus 44 is medium (relative elastic modulus =2). FIG. 7(b) shows-a moment where the bolus 44 fails to deform to the shape of the gullet 13, and the bolus 44, which is out of the gullet 13, is sandwiched between the gullet 13 and the epiglottis 17, and cut into strips. Entrance of the bolus 44, which is cut into pieces, to the respiratory tract 14 causes accidental swallowing and accidental ingestion. That is, even if the bolus 44 is soft to some extent, there is a possibility of a risk of accidental swallowing or accidental ingestion if the bolus 44 cannot deform to a size that enters the gullet 13. FIG. 7(c) illustrates a case where the relative elastic modulus of the bolus 44 is high (relative elastic modulus = 4). Since the bolus 44 has high relative elastic modulus, the shape of the bolus 44 hardly deforms. Obstruction at the epiglottis 17 or a flow to the respiratory tract 14 was able to be estimated.

In the actual phenomenon as well, the experience of choking accidents involving konjac jelly or similar incidents clarifies importance of a size and hardness of a product to prevent an accident of suffocation. Based on a fact that the similar trend was obtained in this simulation result, this suggests a possible use of the simulator using the particle method for the swallowing simulation of a jelly-like bolus.

### (Analysis Case 4)

Some functional deteriorations in a human body probably cause an accidental swallowing and accidental ingestion. A human body was simulated and examined for some functional deteriorations.

Table 2 illustrates simulation conditions of when a movement of the epiglottis 17 became slow. Specifically, an amplitude of movement of the epiglottis 17 was decreased (to the half) in the rotation direction.

**TABLE 2 THE RELATION BETWEEN MOVING PART AND MOVEMENT AMOUNT WHEN A MOVEMENT OF THE EPIGLOTTIS BECAME SLOW**

| MOVING PART | | MOVEMENT AMOUNT | | |
|---|---|---|---|---|
| | | X DIRECTION | Y DIRECTION | ROTATION |
| TONGUE | A | -1*sin(t)-0.5 | 2*sin(t-1) | - |
| | B | -3*sin(t) | -3*sin(t)-1 | - |
| | C | -sin(t-1)-1.0 | -4*sin(t-1) | - |
| | D | -2.0*sin(t)-2.0 | -2*sin(t-3)+2 | - |
| SOFT PALATE | | -2*sin(t) | 0 | 0.5*sin(t-3) |
| EPIGLOTTIS | | sin(t+1.2)-0.8 | -sin(t+1.2)+1 | 0.4*sin(t+1.2)-0.3 |
| GULLET WALL | | 2*sin(t+1.2) | -2*sin(t+1.2) | 0 |

FIG. 8 illustrates the simulation results of the case where the movement of the epiglottis 17 became slow. As seen from comparison with FIG. 4, in FIG. 4, at t_{nd} = 0.48, the epiglottis 17 completely "lids" the respiratory tract 14 to prevent a flow of the liquid 41 to the respiratory tract 14. However, it is recognized that in FIG. 8, where functional restriction is made, the epiglottis 17 does not function and most of the water 41 is accidentally ingested to the respiratory tract 14.

Table 3 illustrates analysis conditions where a movement amount of the entrance portion of the gullet 13 is small. Specifically, a moving speed of the gullet wall that walls the gullet 13 and the respiratory tract 14 is set slow (to the half).

**TABLE 3 THE RELATION BETWEEN MOVING PART AND MOVEMENT AMOUNT WHEN A MOVEMENT AMOUNT OF THE ENTRANCE PORTION OF THE GULLET IS SMALL**

| MOVING PART | | MOVEMENT AMOUNT | | |
|---|---|---|---|---|
| | | X DIRECTION | Y DIRECTION | ROTATION |
| TONGUE | A | -1*sin(t)-0.5 | 2*sin(t-1) | - |
| | B | -3*sin(t) | -3*sin(t)-1 | - |
| | C | -sin(t-1)-1.0 | -4*sin(t-1) | - |
| | D | -2.0*sin(t)-2.0 | -2*sin(t-3)+2 | - |
| SOFT PALATE | | -2*sin(t) | 0 | 0.5*sin(t-3) |
| EPIGLOTTIS | | sin(t+1.2)-0.8 | -sin(t+1.2)+1 | 0.8*sin(t+1.2)-0.6 |
| GULLET WALL | | 1*sin(t+1.2) | -1*sin(t+1.2) | 0 |

FIG. 9 illustrates the simulation results. As seen from comparison with FIG. 4, in FIG. 4, at t_{nd} = 0.48, the epiglottis 17 completely "lids" the respiratory tract 14 to prevent a flow of the liquid 41 to the respiratory tract 14. However, in FIG. 9, where functional restriction is made, the following can be observed. The epiglottis 17 cannot completely close the respiratory tract 14. Then the half of the water 41 flows to the respiratory tract 14, causing accidental ingestion (accidental inspiration). Thus, a functional deterioration causing an accidental swallowing and accidental ingestion can be examined simulatively with simple setting change.

As described above, the simulator allows analysis of a behavior of the various food product or similar product while being swallowed. A three dimensional analysis is required for quantitative examination. However, in either two dimension or three dimension, the particle method is superior in that these swallowing phenomena are revealed. This respect is advantages in the case where the particle method is applied to the swallowing simulator.

FIG. 10 illustrates an exemplary processing flow of the swallowing simulation method according to the first embodiment. First, the oral cavity model 11 formed of oral cavity organs is formed (S010: oral cavity modeling step). Next, an organ property of each of the oral cavity organs in the oral cavity model 11 is set (S020: organ property setting step). Next, a movement of each of the oral cavity organs in the oral cavity model 11 is set (S030: organ movement setting step). Next, the food product or similar product as an analysis target and a physical property of the food product or similar product are set (S040: food product physical property setting step). These setting contents can be freely selected according to the condition. The setting contents are stored to the storage unit 83. Next, the pseudo food products 41 to 44 formed by modeling the food product are input to the oral cavity (S050: input step). The pseudo food products 41 to 44 are input, for example, by dragging the cursor in the oral cavity with the computer mouse by the evaluator. Next, a movement of each of the oral cavity organs and behaviors of the pseudo food products 41 to 44 while being swallowed in the oral cavity model 11 are analyzed using the particle method (S060: movement analysis step). An MSP method, for example, can be used. Next, analysis results obtained in the movement analysis step (S060) are displayed (S070: display step). Next, easiness of eating or easiness of drinking of the food product is evaluated based on the analysis result of the behavior of the pseudo food product 41 while being swallowed (S080: evaluation step). Evaluation is made by the evaluator while viewing the moving screen on the display unit 82. "Good", "poor", a rank, a score, or similar evaluation is input to a cell in an evaluation table displayed on the display unit 82, for example. After the evaluation, the step is returned to the food product physical property setting step (S040), the physical property of the food product is changed and set, and then the subsequent steps are repeatedly performed to the evaluation step. A physical property value to be changed can be freely selected by determination of the evaluator. However, when an appropriate physical property is found at the first trial, the subsequent settings and evaluations may be omitted. Next, the physical property of the food product determined as appropriate in the evaluation step (S080) is determined (S090: physical property determination step). Here, an appropriate physical property range may be indicated, an appropriate physical property may be classified into ranks, or an optimum value may be selected.

Evaluation items are, for example, as follows.
(a) Whether a swallowing, an accidental swallowing or an accidental ingestion risk (the food product adheres to the palate wall and difficult to be peeled off, obstructs the throat or the gullet, or enters the respiratory tract) exists or not
(b) How long is the swallowing period? Is the threshold exceeded?
(c) How much are stress and shear stress applied to the throat wall? Is the threshold exceeded?
(d) Based on (a) to (c), considering correlativity with a sensory evaluation (tasty, exhilarating feeling, or similar feeling) whose data has been obtained separately, easiness of drinking, easiness of eating, difficulty of drinking, and difficulty of eating are evaluated comprehensively

### (Food Product Development)

Next, this embodiment describes an example where simulation is performed while a physical property value is changed, and the simulation result is led to a food product development.

FIG. 11 illustrates an exemplary configuration of the food product development assistance apparatus 200A. The food product prototype result recording unit 83C, a determination production condition recording unit 83D, and a production condition determiner 84 (shown in bold face type in the drawing) are added to the swallowing simulation apparatus 100A in FIG. 2. The food product prototype result recording unit 83C belongs to the storage unit 83. The food product prototype result recording unit 83C records a result of an experimental production performed under an appropriately set production condition that allows the prototype to have a physical property determined as appropriate by the physical property determiner 70. The production condition determiner 84 is disposed in the personal computer PC. The production condition determiner 84 determines a production condition that sets a physical property of the food product or similar product to the physical property determined as appropriate by the physical property determiner 70 based on the prototype result recorded in the food product prototype result recording unit 83C. The determined production condition is recorded in the determination production condition recording unit 83D of the storage unit 83. This clarifies the production condition that sets the physical property of the food product or similar product to the physical property determined as appropriate by the physical property determiner 70, thus the apparatus reliably produces a development food product with easy-to-eat and/or easy-to-drink physical property.

FIG. 12 illustrates an exemplary processing flow of the food product development method. After the exemplary processing flow of the swallowing simulation method in FIG. 10, the food product prototype production step (S110) and the production condition determination step (S120) are added. The food product prototype production step (S110) produces a prototype under an appropriately set production condition so as to allow the prototype to have the physical property determined as appropriate in the physical property determination step (S090). A prototype of, for example, confectionery is produced while combination conditions of raw materials (such as a combination ratio, a stir or the like), baking conditions (such as a temperature, a period, atmosphere or the like), cooling conditions (such as a temperature, a period, atmosphere or the like), dimensions, and/or similar conditions are appropriately set. A result of the experimental production where the obtained physical property is associated with the production condition is recorded in the food product prototype result recording unit 83C of the storage unit 83. The production condition determination step (S120) determines a production condition that sets the physical property of the food product to the physical property determined as appropriate in the physical property determination step (S090) based on the experimental production result in the food product prototype production step (S110), that is, the experimental production result recorded in the food product prototype result recording unit 83C. In the food product prototype production step (S110), for example, if a result where the physical property determined as appropriate has been obtained with a combination ratio A of raw materials and a baking temperature B, the combination ratio A of raw materials and the baking temperature B are determined as production conditions for setting the physical property to the physical property determined as appropriate. The production condition may be one point or may specify a range. The production condition may determine the optimum value or may classify an appropriate range into ranks. This determination may be made by a human, or may be automatically made by the production condition determiner 84 based on the prototype result recorded in the food product prototype result recording unit 83C. The result determined in the production condition determination step (S120) is, for example, recorded in the determination production condition recording unit 83D of the storage unit 83. When the determination is made by a human, the result may be recorded in a notebook.

### (Analysis Case 5)

Analysis case 1 shows an exemplary analysis of a swallowing value experiment with water (assuming 1 mPa•s) being simulated. However, here, analysis is performed while a degree of viscosity of a fluid is changed. Vegetable oil such as olive oil, olive squalane, castor oil, carnauba wax or the like as emulsifier can be added to, for example, a water phase. Combining these oily ingredients to be 0.5 to 20 weight percent of the entire liquid forms an emulsified solution, which is an oil-in-water type of emulsion. This allows adjusting a degree of viscosity of the emulsified solution. Additionally, viscosity can be adjusted by rotation speed of stir (for example, see Japanese Patent Application Publication No. 10-182339).

Using the emulsified solution with the degree of viscosity thus being changed as a pseudo food product, an analysis is performed using a swallowing simulator. A swallowing period t_{nd} is probably increased with increasing degree of viscosity. A threshold is set for the swallowing period. A swallowing period equal to or less than the threshold is regarded as appropriate while a swallowing period exceeding the threshold is regarded inappropriate. Thus, appropriate degree of viscosity range can be determined. Accordingly, in the food product prototype production step (S110), a prototype is produced with setting, for example, a combination ratio of emulsifier or/and a rotation speed of stir of emulsifier as a production condition. Thus, an appropriate production condition is determined through experimental production.

### (Analysis Case 6)

Analysis case 2 changes adhesiveness of the bolus 42 for analysis. Now, a rice cake made from glutinous rice is a representative example of a bolus with high adhesiveness. However, waxy wheat has been developed by Ministry of Agriculture, Forestry and Fisheries (excerpted from the website of the Waxy Wheat Product Development Study Group, [online], [searched on June 29, 2011], entitled "What is waxy wheat?" http://mochikomugi.com/hiroba (Reference Material 1) and "FAQ," http://mochikomugi.com/faq (Reference Material 2). A rice cake made from the waxy wheat is said to be low adhesiveness compared with a rice cake made from rice and therefore features easiness of eating. Therefore, adhesiveness of a rice cake can be probably changed by mixing glutinous rice with waxy wheat and changing their mixing ratio. Additionally, changing a period during which the rice cake is kneaded can probably change the adhesiveness. A swallowing simulation is performed on a pseudo food product with adhesiveness being changed. In the case where the pseudo food product adheres to the palate wall or the soft palate and is difficult to be peeled off, or blocks the throat or the gullet while being swallowed, or a swallowing period exceeds the threshold, the case is determined as inappropriate. Meanwhile, cases where these situations do no occur are determined as appropriate. This allows determining an appropriate adhesiveness range. Accordingly, in the food product prototype production step (S110), a prototype is produced with setting, for example, a combination ratio of glutinous rice and waxy wheat or a period of kneading a rice cake as a production condition. Thus, an appropriate production condition is determined through experimental production.

### (Analysis Case 7)

The analysis case 2 simulates that the adherent bolus 42 obstructing near the soft palate 16 is washed away using rinse water 43. Here, a simulation that the adherent bolus 42 is washed away using the emulsified solution where viscosity is changed in the analysis case 5 is performed. Nonetheless, in the case where the pseudo food product adheres to the palate wall and/or the soft palate and is difficult to be peeled off, blocks the throat and the gullet, enters the respiratory tract, or a swallowing period exceeds the threshold during the swallowing simulation, the case is determined as inappropriate. Meanwhile, cases where these situations do no occur are determined as appropriate. This allows determining an appropriate degree of viscosity range. Accordingly, in the food product prototype production step (S110), a prototype is produced with setting, for example, a combination ratio of emulsifier and/or a rotation speed of stir as a production condition. Thus, an appropriate production condition is determined through experimental production.

### (Analysis Case 8)

The analysis case 3 performs the swallowing simulation on the bolus with elastic modulus such as a jelly. Here, simulations are performed while a relative elastic modulus is widely changed. In the case where the torn bolus 44 enters the respiratory tract 14 or the bolus 44 cannot be deformed to a size where the bolus 44 can enter the gullet 13, the case is determined as inappropriate. Meanwhile, cases where these situations do no occur are determined as appropriate. This allows determining an appropriate elastic modulus range. In the jelly type food/drink product, for example, in the case where a gelatinizing agent such as carrageenan, xanthan gum, locust bean gum, pectin, mannan or the like is combined so as to be 0.001 to 1.0 weight percent of overall weight of the jelly type food/drink product, the storage elastic modulus changes between 0.05 and 150 Pa (see, for example, Japanese Patent Application Publication No. 2001-299241). Accordingly, in the food product prototype production step (S110), to make the elastic modulus of the jelly type food/drink product appropriate, the prototype is produced with setting, for example, the combination amount of the gelling agent as a production condition. Thus, an appropriate production condition is determined through experimental production.

### (Analysis Case 9)

Analysis case 3 performs the swallowing simulation on the bolus with elastic modulus such as jelly. Here, simulations are performed while a dimension is changed. The simulations are performed while, for example, an initial diameter of the jelly type food/drink product is changed between 2 mm and 20 mm. In the case where the torn bolus 44 enters the respiratory tract 14 or the bolus 44 cannot be deformed to a size where the bolus 44 can enter the gullet 13, the case is determined as inappropriate. Meanwhile, cases where these situations do no occur are determined as appropriate. This allows determining an appropriate dimension range. The dimensions are prepared by dimensions of a mold form of a container. In the food product prototype production step (S110), a prototype is produced with setting, for example, the dimensions of the mold form of the container that solidifies jelly as a production condition. Then, an appropriate production condition is determined through experimental production.

### (Food Product Production Method)

FIG. 13 illustrates an exemplary processing flow of the food product production method. The food product production step includes a food product preparation step (S130). The food product preparation step (S130) prepares a food product using the production condition determined as the production condition that sets the physical property determined as appropriate in the physical property determination step (S090) in the production condition determination step (S120) of the food product development method in FIG. 12. The food product preparation step (S130) may be performed in any step in the production steps. In production of confectionery the food product or similar product can be prepared in a raw material combination step, a baking step, or the like. Then, in the case where the production condition in this food product preparation step (S130) is determined in the production condition determination step (S120), this corresponds to the embodiment. The result determined in the production condition determination step (S120) is recorded, for example, in the determination production condition recording unit 83D of the storage unit 83.

As described above, according to the embodiment, the organ properties, the movements of the oral cavity organs, and the physical property of the food product are set to the oral cavity model 11. Then, the behavior of the food product is analyzed using the particle method. This allows to analyze a phenomenon of swallowing using the swallowing simulation method that facilitates reproduction of the actual phenomenon of swallowing and further to develop food products which are easy-to-eat or easy-to-drink.

### (Second embodiment)

In the first embodiment, an exemplary swallowing evaluation made by inputting the food product and viewing the moving image by the evaluator is described. In the second embodiment, an example where the swallowing simulation apparatus automatically inputs the food product based on the setting and automatically performs the swallowing evaluation is described. The following mainly describes the points different from the first embodiment (similarly, in the following embodiments, the points different from an antecedent embodiment are mainly described).

FIG. 14 illustrates an exemplary configuration of the swallowing simulation apparatus 100B according to the second embodiment. An evaluation unit 60, the pseudo screen display unit 82A, and the food product input setting unit 45 are added in the personal computer (PC) compared with the first embodiment (see FIG. 2). The evaluation unit 60 automatically evaluates easiness of eating or easiness of drinking of the food product. The pseudo screen display unit 82A displays the analysis result of the behavior of the pseudo food product while being swallowed on the virtual moving screen. The food product input setting unit 45 sets an input condition of the pseudo food product. The evaluation condition storage unit 83A is added in the storage unit 83. The evaluation condition storage unit 83A stores an evaluation condition. Other configurations are same to the first embodiment.

FIG. 15 illustrates an exemplary processing flow of the swallowing simulation method. A food product input setting step (S045) is added before the input step (S050) compared with the first embodiment (see FIG. 10). The food product input setting step (S045) sets the input condition of the food product. The display step (S070) for displaying the moving screen on the display unit 82 is replaced by the step for displaying the virtual moving screen on the pseudo screen display unit 82A (S075). In the evaluation step (S080), the evaluation unit 60 makes an automatic evaluation. Other steps are same to the first embodiment.

In the second embodiment, an injection position and injection timing of the food product are preset to the food product input setting unit 45 (S045: food product input setting step). The injection position of the pseudo food product in the oral cavity is, for example, set near the teeth in the oral cavity (for example, within 1/2 length of the pseudo food product). Next, the pseudo food product is injected in the oral cavity in accordance with the setting conditions (position and timing) (S050: input step). For automatic evaluation, the evaluation condition is preliminarily stored to the evaluation condition storage unit 83A. The behaviors of the oral cavity model 11 and the pseudo food product 41 as the analysis results of the simulations are displayed on the virtual moving screen of the pseudo screen display unit 82A in the personal computer PC. The display of the pseudo screen display unit 82A is collated with the evaluation condition of the evaluation condition storage unit 83A by the evaluation unit 60. Thus, evaluation is performed.

Evaluation items are, for example, as follows.
(a) Whether the swallowing, the accidental swallowing or accidental ingestion risk (the food product adheres to the palate wall or the like and difficult to be peeled off, obstructs the throat or the gullet, or enters the respiratory tract) exists or not
(b) How long is the swallowing period? Is the threshold exceeded?
(c) How much are stress or shear stress applied to the throat wall? Is the threshold exceeded?
(d) Based on (a) to (c), considering correlativity with a sensory evaluation (tasty, exhilarating feeling, or similar feeling) whose data has been obtained separately, easiness of drinking, easiness of eating, difficulty of drinking, or difficulty of eating is evaluated comprehensively. (a) to (c) and the sensory evaluations are preliminarily converted into values, respectively. Then, the values are multiplied by a weighting factor and are summed. The total is automatically and comprehensively evaluated, (c) and the sensory evaluation may be omitted.

FIG. 16 illustrates an exemplary configuration of the food product development assistance apparatus 200B. The food product prototype result recording unit 83C, the determination production condition recording unit 83D, and the production condition determiner 84 (shown in bold face type in the drawing) are added to the swallowing simulation apparatus 100B in FIG. 14. The food product prototype result recording unit 83C records a result of an experimental production performed under an appropriately set production condition that allows the prototype to have a physical property determined as appropriate by the physical property determiner 70. The production condition determiner 84 determines a production condition that sets a physical property of the food product or similar product to the physical property determined as appropriate by the physical property determiner 70 based on the prototype result recorded in the food product prototype result recording unit 83C. The determined production condition is recorded in the determination production condition recording unit 83D of the storage unit 83.

FIG. 17 illustrates an exemplary processing flow of the food product development method. After the exemplary processing flow of the swallowing simulation method in FIG. 15, the food product prototype production step (S110) and the production condition determination step (S120) are added. This clarifies the production condition that sets a physical property to the physical property determined as appropriate by the physical property determiner 70, thus the method reliably produces a development food product with easy-to-eat and/or easy-to-drink physical property.

Other configurations and processing flows are same to the first embodiment. Similarly to the first embodiment, this allows to analyze a phenomenon of swallowing using the swallowing simulation method that facilitates reproduction of the actual phenomenon of swallowing and further to develop food products which are easy-to-eat or easy-to-drink.

Additionally, even the case where one of the input and the evaluation is performed by the human and the other is performed by the computer is similarly applicable and similar effects can be achieved.

### (Third embodiment)

While in the above described embodiments, an example of evaluation by the evaluator and an example of automatic evaluation by the swallowing simulation apparatus are described, the evaluation result (partial or overall evaluation result) may be displayed on the swallowing simulation apparatus for requesting the evaluator to evaluate. Regarding a processing flow, in the evaluation step (S080) of the second embodiment, the evaluation result (partial or overall evaluation result) by the swallowing simulation apparatus is displayed on the display unit 82 together with the evaluation table. With reference to the evaluation result by the swallowing simulation apparatus, the evaluator inputs his/her evaluation result on the evaluation table. The input step (S050) may be manually input by the human or may be automatically input. Other apparatus configurations and processing flows are same to the second embodiment. Similarly to the second embodiment, the swallowing simulation apparatus and the swallowing simulation method that facilitate reproduction of the actual phenomenon of swallowing can be provided.

### (Fourth embodiment)

In the above described embodiments, the physical property of the food product or similar product automatically determined as appropriate by the physical property determiner 70 is described as an example. In this embodiment, an exemplary determination made by the human is described. In the apparatus configuration of the embodiment, typically, the physical property determiner 70 in FIG. 2 of the first embodiment is removed. FIG. 10 can be used as an exemplary processing flow. The physical property determiner 70 may be present. In this case, the physical property determiner 70 is not used or a determination result by the physical property determiner 70 is shown to a decider (for example, the evaluator) as a reference. The physical property of the food product or similar product is determined as appropriate by the human. However, there is no difference in that the determination is made based on the evaluation result. Although, the determination is possibly slightly changed in an intellectual process, almost similar results are predicted. Other apparatus configurations and processing flows are same to the first embodiment. Similarly to the first embodiment, the swallowing simulation apparatus and the swallowing simulation method that facilitate reproduction of the actual phenomenon of swallowing can be provided. When a determination is made by the human, this applies to the second embodiment and the third embodiment similarly to the first embodiment.

### (Fifth embodiment)

This embodiment describes an example of applying the swallowing simulator according to the present invention to dietary education.

FIG. 18 illustrates an exemplary configuration of the dietary education assistance apparatus 300A in this embodiment. Compared with the swallowing simulation apparatus 100A in the first embodiment (see FIG. 2), the teaching unit 85 is added while the physical property determiner 70 is removed. The teaching unit 85 teaches a behavior of the pseudo food product 41 while being swallowed displayed on the moving screen by the display unit 82 associating with the evaluation result of the food product recorded in the evaluation result recording unit 83B. In this embodiment, the teaching contents are based on the evaluation result of the food product recorded in the evaluation result recording unit 83B. The interpretation contents are preliminary created and recorded. The teaching unit 85 may automatically create the interpretation contents based on the evaluation result. However, it is preferred that the interpretation contents be edited and supplemented for easier understanding by an educator. Additionally, in this embodiment, the physical property determiner 70 is removed. However, the physical property determiner 70 may be included so that an educator may interpret about determination of a physical property. The teaching unit 85 stores the interpretation contents. The teaching unit 85 also displays analysis results of movement of each of the oral cavity organs and the behavior of the pseudo food product 41 while being swallowed analyzed by the movement analysis unit 50 on the moving screen of the display unit 82. The interpretation contents are phonetically output to, for example, a speaker of the display unit 82.

FIG. 19 illustrates an exemplary processing flow of the dietary education method according to this embodiment. Compared with the simulation method in the first embodiment (see FIG. 10), the teaching step (S082) is added after the evaluation step (S080), and the physical property determination step (S090) is removed. The teaching step (S082) teaches a behavior of the pseudo food product while being swallowed displayed on the moving screen in the display step (S070) associating with the evaluation result of the food product evaluated in the evaluation step (S080). In this embodiment, the physical property determination step (S090) is removed. However, the physical property determination step (S090) may be included so that an educator may interpret about determination of a physical property. Accordingly, the swallowing phenomenon is displayed using the swallowing simulation method, which facilitates the reproduction of actual phenomenon of swallowing. Accordingly, easiness of eating or easiness of drinking of the food product are easily understood, and the method is effective in dietary education.

### (Program)

The present invention is also applicable as a program readable by the computer to make the computer execute the above described swallowing simulation methods. Additionally, the present invention can be achieved as a storage medium to store the program. The program may be stored to the controller of the swallowing simulation apparatus for use, may be stored to the built-in or external storage device for use, or may be downloaded from the Internet for use.

The preferred embodiments of the present invention are described above. However, the present invention should not be limited to these embodiments. Various improvements are possible without departing from the scope of the present invention.

While in the above described embodiments, for example, exemplary movements of the moving wall of the tongue, the soft palate, the epiglottis, and the gullet wall are described, the motion equation and the parameter can be freely changed. Additionally, a movement can be given to other than the above described four organs, for example, a tooth. Thus, an influence of mastication to the swallowing can be reflected. The exemplary food products are up to two. However, the three or more food products can be operated together and the behavior can be analyzed. Further, an analysis of solids with mutually different physical property value, for example, chocolate covering peanuts (solid-solid), an analysis of chocolate incorporating liqueur (solid-liquid), and also an analysis of mixed liquid of liquid (liquid-liquid) with mutually different physical property value, for example, dressing (oil and vinegar) are possible. Besides, the details can be variously changed, for example, the organs and the food products can be displayed in different colors.

Additionally, in the above described embodiments, for example, exemplary preparations of degree of viscosity, adhesiveness, and elastic modulus of the food product are described. Besides, simulation may be performed while dimensions of peanut chocolate, a thickness of a chocolate layer, or the like is changed to determine an appropriate physical property so as to perform preparation of the food product.

### Industrial Applicability

The present invention is used for development of a food product using a swallowing simulation for food and drink.

Use of the terms "a," "an," "the" and similar referents used in the context in explanation of the invention (particularly in the context of claims as described below) is to be construed to cover both the singular form and the plural form, unless otherwise indicated herein or clearly contradicted by context. The terms "comprising," "having," "including" and "containing" are to be construed as open-ended terms (more specifically, meaning "including, but not limited to") unless otherwise noted. Recitation of ranges of values herein are merely intended to serve as a shorthand method of referring individually to each separate value falling within the range, unless otherwise indicated herein, and each separate value is incorporated herein as if it were individually recited herein. All methods described herein can be performed in any suitable order unless otherwise indicated herein or otherwise clearly contradicted by context. The use of any and all examples, or exemplary language ("such as") provided herein, is intended merely to better illuminate the invention and does not pose a limitation on the scope of the invention unless otherwise claimed. No language herein should be construed as indicating any non-claimed element as essential to the practice of the invention.

Preferred embodiments of the invention are described herein, including the best mode known to the present inventors for carrying out the present invention. Variations of the preferred embodiments may become apparent to those skilled in the art upon reading the foregoing description. The present inventors expect skilled artisans to employ such variations as appropriate, and the present inventors intend for the invention to be practiced otherwise than as specifically described herein.

### [Description of Reference Numerals and Symbols]

- 10: oral cavity modeling unit
- 11: oral cavity model
- 12: oral cavity wall
- 13: gullet
- 14: respiratory tract
- 15: tongue
- 16: soft palate
- 17: epiglottis
- 18: moving wall
- 19: gullet wall
- 20: organ property setting unit
- 30: organ movement setting unit
- 40: food product physical property setting unit
- 41 to 44, 49: pseudo food product
- 45: food product input setting unit
- 50: movement analysis unit
- 60: evaluation unit
- 70: physical property determiner
- 75: organ movement determiner
- 81: input unit
- 82: display unit
- 82A: pseudo screen display unit
- 83: storage unit
- 83A: evaluation condition storage unit
- 83B: evaluation result recording unit
- 83C: food product prototype result recording unit
- 83D: determination production condition recording unit
- 84: production condition determiner
- 85: teaching unit
- 90: controller
- 100A, 100B, 100C: swallowing simulation apparatus
- 200A, 200B: food product development assistance apparatus
- 300A: dietary education assistance apparatus
- PC: personal computer
- t_{nd}: dimensionless swallowing period

## Claims

1. A food product development assistance apparatus (200B) for developing an easy-to-eat or easy-to-drink food product, a medicinal product or a quasi-drug, comprising;
an oral cavity modeling unit (10) configured to form a two dimensional oral cavity model (11) formed of oral cavity organs using a particle method, wherein a behavior of a pseudo food product (41, 42, 43) in the oral cavity model (11) is analyzed using organ properties, movements of the oral cavity organs and a physical property of the pseudo food product (41, 42, 43) set so that reproduction of actual phenomenon of swallowing is facilitated;
a storage unit (83) configured to record the oral cavity model (11);
wherein the oral cavity model (11) includes an oral cavity wall (12), a gullet (13), a respiratory tract (14), a tongue (15), a soft palate (16) and an epiglottis (17); the tongue (15), the soft palate (16), the epiglottis (17) and a gullet (13) entrance are set as elastic bodies while the others are set as rigid bodies, a movement of the tongue (15) is expressed by a wave movement, movements of the soft palate (16) and the epiglottis (17) are expressed by reciprocations at bases and rotational movement around the bases, and a movement of an entrance portion of the gullet (13) is expressed by reciprocation in a perpendicular direction to a central axis of the gullet (13), moving walls are embedded in the tongue (15) and the movement of the tongue (15) is performed while changing an amplitude of oscillation at same period and shifting phase;
the apparatus further comprising;
an organ property setting unit (20) configured to set organ property of each of the oral cavity organs in the oral cavity model (11) stored in the storage unit (83);
an organ movement setting unit (30) configured to set a movement of each of the oral cavity organs in the oral cavity model (11);
a food product physical property setting unit (40) configured to set a food product, a medicinal product or a quasi-drug as an analysis target and a physical property of the analysis target;
an input unit (81) configured to input a pseudo food product (41, 42, 43) to the oral cavity in the two dimensional oral cavity model (11), the pseudo food product (41, 42, 43) being formed by modeling the analysis target;
a movement analysis unit (50) configured to analyze a movement of each of the oral cavity organs and a behavior of the pseudo food product (41, 42, 43) while being swallowed in the oral cavity model (11) using the organ properties set by the organ property setting unit (20), the movements of the oral cavity organs set by the organ movement setting unit (30), and the physical property of the food product set by the food product physical property setting unit (40) and using the particle method;
wherein the storage unit (83) configured to record an analysis result of the movement of each of the oral cavity organs and the behavior of the pseudo food product (41, 42, 43);
the apparatus, further comprising;
a display unit (82A) configured to display, on a virtual moving screen formed at a virtual space of the food product development assistance apparatus (200B), the analysis result of the movement of each of the oral cavity organs and the behavior of the pseudo food product (41, 42, 43) while being swallowed on a moving screen, the analysis result being analyzed by the movement analysis unit (50);
an evaluation unit (60) configured to evaluate easiness of eating and/or easiness of drinking of the analysis target based on the behavior of the pseudo food product (41, 42, 43) while being swallowed on the virtual moving screen, wherein evaluation unit (60) is adapted to automatically evaluate whether the behavior of the pseudo food product (41, 42, 43) on the virtual moving screen meets a predetermined condition or not, wherein said predetermined condition is at least one of the conditions: whether the pseudo food product (41, 42, 43) enters the respiratory tract, whether the pseudo food product (41, 42, 43) gets stuck to the gullet (13), whether the pseudo food product (41, 42, 43) adheres to the tongue (15) or the gullet (13), whether a period from introduction of the pseudo food product (41, 42, 43) in the oral cavity to passing it through the gullet (13) is within a predetermined range, whether stress applied by the pseudo food product (41, 42, 43) to a wall surface is equal to or less than a predetermined value, whether shear stress caused by the pseudo food product (41, 42, 43) at the wall surface is equal to or less than a predetermined value;
wherein the storage unit (83) has an evaluation result recording unit (83B) configured to record an evaluation result of easiness of eating or easiness of drinking of the analysis target evaluated by the evaluation unit (60) ;
the apparatus, further comprising:
a physical property determiner (70) configured to determine the physical property of the analysis target regarded as appropriate based on the evaluation results recorded repeatedly in the evaluation result recording unit (83B); wherein analyzing by the movement analysis unit (50), evaluation by the evaluation unit (60) and recording by the evaluation results recording unit are repeatedly performed when the physical property of the food product is changed and set by the food product physical property setting unit (40);s
wherein the storage unit (83) has a food product prototype result recording unit configured to record a result of an experimental production performed under an appropriately set production condition to have the physical property determined as appropriate by the physical property determiner (70); and
the apparatus, further comprising;
a production condition determiner (84) configured to determine a production condition that sets a physical property of the analysis target to the physical property determined as appropriate by the physical property determiner (70) based on a prototype result recorded in the food product prototype result recording unit (83C), wherein the results of experimental production are recorded when the production conditions of experimental productions are changed;
wherein, the storage unit (83) has a determination production condition recording unit (83D) configured to record a result determined by the production condition determiner (84).

2. The food product development assistance apparatus (200B) according to claim 1, wherein at least three moving walls are embedded in the tongue (15).

3. A food product development method to be performed by a food product development assistance apparatus (200B) for developing an easy-to-eat or easy-to-drink food product, a medicinal product or a quasi-drug, the method comprising:
an oral cavity modeling step of forming a two dimensional oral cavity model (11) formed of oral cavity organs in a two dimensional space using a particle method, wherein a behavior of a pseudo food product (41, 42, 43) in the oral cavity model (11) is analyzed using organ properties, movements of the oral cavity organs and a physical property of the pseudo food product (41, 42, 43) set so that reproduction of actual phenomenon of swallowing is facilitated;
an oral cavity model (11) recording step of recording the oral cavity model (11);
wherein the oral cavity model (11) includes an oral cavity wall (12), a gullet (13), a respiratory tract (14), a tongue (15), a soft palate (16) and an epiglottis (17); the tongue (15), the soft palate (16), the epiglottis (17) and a gullet (13) entrance are set as elastic bodies while the others are set as rigid bodies, a movement of the tongue (15) is expressed by a wave movement, movements of the soft palate (16) and the epiglottis (17) are expressed by reciprocations at bases and rotational movement around the bases, and a movement of an entrance portion of the gullet (13) is expressed by reciprocation in a perpendicular direction to a central axis of the gullet (13), moving walls are embedded in the tongue (15) and the movement of the tongue (15) is performed while changing an amplitude of oscillation at same period and shifting phase;
the method further comprising;
an organ property setting step of setting an organ property of each of the oral cavity organs in the oral cavity model (11) stored in the oral cavity model (11) recording step;
an organ movement setting step of setting a movement of each of the oral cavity organs in the oral cavity model (11);
a food product physical property setting step of setting a food product or similar product as an analysis target and a physical property of the analysis target;
an input step of inputting a pseudo food product (41, 42, 43) to the oral cavity in the two dimensional, the pseudo food product (41, 42, 43) being formed by modeling the analysis target;
a movement analysis step of analyzing a movement of each of the oral cavity organs and a behavior of the pseudo food product (41, 42, 43) while being swallowed in the oral cavity model (11) using the organ properties set in the organ property setting step, the movements of the oral cavity organs set in the organ movement setting step, and the physical property of the food product set in the food product physical property setting step and using the particle method;
an analysis result recording step of recording an analysis result of the movement of each of the oral cavity organs and the behavior of the pseudo food product (41, 42, 43);
a display step of displaying, on a virtual moving screen formed at a virtual space of the food product development assistance apparatus (200B), the analysis result of the movement of each of the oral cavity organs and the behavior of the pseudo food product (41, 42, 43) while being swallowed on a moving screen, the analysis result being analyzed in the movement analysis step;
an evaluation step of evaluating easiness of eating or easiness of drinking of the analysis target based on the behavior of the pseudo food product (41, 42, 43) while being swallowed on the virtual moving screen, wherein the evaluation step automatically evaluates whether the behavior of the pseudo food product (41, 42, 43) on the virtual moving screen meets a predetermined condition or not, wherein said predetermined condition is at least one of the conditions: whether the pseudo food product (41, 42, 43) enters the respiratory tract, whether the pseudo food product (41, 42, 43) gets stuck to the gullet (13), whether the pseudo food product (41, 42, 43) adheres to the tongue (15) or the gullet (13), whether a period from introduction of the pseudo food product (41, 42, 43) in the oral cavity to passing it through the gullet (13) is within a predetermined range, whether stress applied by the pseudo food product (41, 42, 43) to a wall surface is equal to or less than a predetermined value, whether shear stress caused by the pseudo food product (41, 42, 43) at the wall surface is equal to or less than a predetermined value;
an evaluation result recording step of recording an evaluation result of easiness of eating or easiness of drinking of the analysis target of said evaluation step;
a physical property determination step of determining the physical property of the analysis target regarded as appropriate based on the evaluation results recorded repeatedly in the evaluation result recording step, wherein the food product physical property setting step, the movement analysis step, the evaluation step and the evaluation result recording step are repeatedly performed when the physical property of the food product is changed and set in the food product physical property setting step;
a food product prototype production step of performing an experimental production under an appropriately set production condition to have the physical property determined as appropriate in the physical property determination step;
a food product prototype result recording step of recording a result of an experimental production performed under an appropriately set production condition to have the physical property determined as appropriate by the physical property determiner (70);
a production condition determination step of determining a production condition that sets a physical property of the analysis target to the physical property determined as appropriate in the physical property determination step based on the prototype result in the food product prototype production step, wherein the results of experimental production are recorded and compared when the production conditions of experimental productions are changed; and
a determination production condition recording step of recording a result determined by the production condition determination step.

4. The food product development method according to claim 3; wherein at least three moving walls are embedded in the tongue (15).

5. The food product development method according to claim 3 or 4; wherein
the method repeatedly performs from the food product physical property setting step to the evaluation step while the physical property of the analysis target is changed and set in the food product physical property setting step.

6. The food product development method according to one of claims 3 to 5; wherein
the organ property setting step sets an oral cavity wall (12) as a rigid body and a tongue (15) as an elastic body;
the organ movement setting step sets a plurality of moving walls in the tongue (15), the tongue (15) being set so as to move in a wave movement by moving the plurality of moving walls to a direction intersecting with a surface of the tongue (15) with a predetermined period and a predetermined phase difference, and sets a soft palate (16), an epiglottis (17), and a gullet (13) wall so as to move together with a predetermined phase difference to the wave movement; and
the movement analysis step treats the tongue (15) and the pseudo food product (41, 42, 43) as particles.

7. The food product development method according to one of claims 3 to 6; wherein
the organ movement setting step sets a movement of a soft palate (16) and an epiglottis (17) as a movement of a rotator where a rotational center moves.

8. The food product development method according to one of claims 3 to 7; wherein
the food product physical property setting step sets a plurality of liquid, semisolid, or solid pseudo food products (41, 42, 43) with different physical property as an analysis target; and
the movement analysis step determines free surfaces of a plurality of the pseudo food products (41, 42, 43) and boundaries between the plurality of pseudo food products (41, 42, 43), the movement analysis step analyzing a gearing behavior of the plurality of pseudo food products (41, 42, 43).

9. The food product development method according to one of claims 3 to 8; wherein
the oral cavity modeling step forms a two dimensional oral cavity model (11); and
the movement analysis step analyzes the behavior of the pseudo food product (41, 42, 43) in a two dimensional space.

10. A food product production method, comprising the steps of the method according to one of claims 3 to 9; and
a food product preparation step of preparing the food product using a production condition determined as appropriate in the production condition determination step for allowing the food product to have the physical property that is determined as appropriate.

## Patentansprüche

1. Assistenzvorrichtung (200B) zur Lebensmittelproduktentwicklung für die Entwicklung eines einfach zu essenden oder einfach zu trinkenden Lebensmittelprodukts, eines medizinischen Produkts oder eines Quasi-Medikaments, umfassend:
eine Mundhöhlenmodellierungseinheit (10), die eingerichtet ist, um ein aus Mundhöhlenorganen gebildetes zweidimensionales Mundhöhlenmodell (11) unter Verwendung eines Teilchenverfahrens zu bilden, wobei ein Verhalten eines Pseudo-Lebensmittelprodukts (41, 42, 43) in dem Mundhöhlenmodell (11) unter Verwendung von Organeigenschaften, Bewegungen der Mundhöhlenorgane und einer physikalischen Eigenschaft des Pseudo-Lebensmittelprodukts (41, 42, 43) analysiert wird, die derart eingestellt sind, dass eine Reproduktion der tatsächlichen Erscheinung des Schluckens erleichtert wird;
eine Speichereinheit (83), die eingerichtet ist, um das Mundhöhlenmodell (11) aufzuzeichnen;
wobei das Mundhöhlenmodell (11) eine Mundhöhlenwand (12), eine Speiseröhre (13), Atemwege (14), eine Zunge (15), einen Gaumensegel (16) und einen Kehldeckel (17) enthält; wobei die Zunge (15), der Gaumensegel (16), der Kehldeckel (17) und ein Eingang der Speiseröhre (13) als elastische Körper eingestellt sind, während die anderen als starre Körper eingestellt sind, eine Bewegung der Zunge (15) durch eine Wellenbewegung ausgedrückt wird, Bewegungen des Gaumensegels (16) und des Kehldeckels (17) durch Hin- und Herbewegungen an den Basen und eine Rotationsbewegung um die Basen ausgedrückt werden und eine Bewegung eines Eingangsabschnitts der Speiseröhre (13) durch eine Hin- und Herbewegung in einer zu einer Zentralachse der Speiseröhre (13) senkrechten Richtung ausgedrückt wird, sich bewegende Wände in der Zunge (15) eingebettet sind und die Bewegung der Zunge (15) durchgeführt wird, während eine Schwingungsamplitude bei gleicher Phase und Phasenverschiebung geändert wird;
die Vorrichtung ferner umfassend:
eine Organeigenschaft-Einstellungseinheit (20), die eingerichtet ist, um eine Organeigenschaft jedes der Mundhöhlenorgane in dem Mundhöhlenmodell (11), das in der Speichereinheit (83) gespeichert ist, einzustellen;
eine Organbewegungs-Einstellungseinheit (30), die eingerichtet ist, um eine Bewegung jedes der Mundhöhlenorgane in dem Mundhöhlenmodell (11) einzustellen;
eine Einstellungseinheit (40) für eine physische Eigenschaft eines Lebensmittelprodukts, die eingerichtet ist, um ein Lebensmittelprodukt, ein medizinisches Produkt oder ein Quasi-Medikament als ein Analyseobjekt und eine physikalische Eigenschaft des Analyseobjekts einzustellen;
eine Eingabeeinheit (81), die eingerichtet ist, um ein Pseudo-Lebensmittelprodukt (41, 42, 43) in die Mundhöhle in dem zweidimensionalen Mundhöhlenmodell (11) einzugeben, wobei das Pseudo-Lebensmittelprodukt (41, 42, 43) durch Modellierung des Analyseobjekts gebildet wird;
eine Bewegungsanalyseeinheit (50), die eingerichtet ist, um eine Bewegung jedes der Mundhöhlenorgane und ein Verhalten des Pseudo-Lebensmittelprodukts (41, 42, 43) zu analysieren, während es in dem Mundhöhlenmodell (11) geschluckt wird, unter Verwendung der Organeigenschaften, die durch die Organeigenschaft-Einstellungseinheit (20) eingestellt werden, der Bewegungen der Mundhöhlenorgane, die durch die Organbewegung-Einstellungseinheit (30) eingestellt werden, und der physikalischen Eigenschaft des Lebensmittelprodukts, die durch die Einstellungseinheit (40) für eine physische Eigenschaft eines Lebensmittelprodukts eingestellt wird, und unter Verwendung des Teilchenverfahrens;
wobei die Speichereinheit (83) eingerichtet ist, um ein Analyseergebnis der Bewegung jedes der Mundhöhlenorgane und des Verhaltens des Pseudo-Lebensmittelprodukts (41, 42, 43) aufzuzeichnen;
die Vorrichtung ferner umfassend:
eine Anzeigeeinheit (82A), die eingerichtet ist, um auf einem virtuellen Bewegungsbildschirm, der in einem virtuellen Raum der Assistenzvorrichtung (200B) zur Lebensmittelproduktentwicklung gebildet ist, das Analyseergebnis der Bewegung jedes der Mundhöhlenorgane und des Verhaltens des Pseudo-Lebensmittelprodukts (41, 42, 43), während es auf einem Bewegungsbildschirm geschluckt wird, anzuzeigen, wobei das Analyseergebnis von der Bewegungsanalyseeinheit (50) analysiert wird;
eine Auswertungseinheit (60), die eingerichtet ist, um die Einfachheit des Essens und/oder die Einfachheit des Trinkens des Analyseobjekts basierend auf dem Verhalten des Pseudo-Lebensmittelprodukts (41, 42, 43), während es auf dem virtuellen Bewegungsbildschirm geschluckt wird, auszuwerten, wobei die Auswertungseinheit (60) ausgelegt ist, um automatisch auszuwerten, ob das Verhalten des Pseudo-Lebensmittelprodukts (41, 42, 43) auf dem virtuellen Bewegungsbildschirm mit einem vorbestimmten Zustand übereinstimmt oder nicht, wobei der vorbestimmte Zustand mindestens einer der folgenden Zustände ist: ob das Pseudo-Lebensmittelprodukt (41, 42, 43) in die Atemwege eintritt, ob das Pseudo-Lebensmittelprodukt (41, 42, 43) in der Speiseröhre (13) stecken bleibt, ob das Pseudo-Lebensmittelprodukt (41, 42, 43) an der Zunge (15) oder der Speiseröhre (13) klebt, ob ein Zeitraum von der Einführung des Pseudo-Lebensmittelprodukts (41, 42, 43) in die Mundhöhle bis zum Passieren dieses durch die Speiseröhre (13) innerhalb eines vorbestimmten Bereichs liegt, ob Druck, der durch das Pseudo-Lebensmittelprodukt (41, 42, 43) auf eine Wandfläche ausgeübt wird, kleiner gleich einem vorbestimmten Wert ist, ob Scherspannung, die durch das Pseudo-Lebensmittelprodukt (41, 42, 43) an der Wandfläche verursacht wird, kleiner gleich einem vorbestimmten Wert ist;
wobei die Speichereinheit (83) eine Auswertungsergebnisaufzeichnungseinheit (83B) aufweist, die eingerichtet ist, um ein Auswertungsergebnis der Leichtigkeit des Essens oder der Leichtigkeit des Trinkens des Analyseobjekts, das von der Auswertungseinheit (60) ausgewertet wird, aufzuzeichnen;
die Vorrichtung ferner umfassend:
einen Physikalische-Eigenschaft-Bestimmer (70), der eingerichtet ist, um die physikalische Eigenschaft des Analyseobjekts, die als geeignet basierend auf den Auswertungsergebnissen, die in der Auswertungsergebnisaufzeichnungseinheit (83B) wiederholt aufgezeichnet werden, betrachtet wird, zu bestimmen; wobei das Analysieren durch die Bewegungsanalyseeinheit (50), die Auswertung durch die Auswertungseinheit (60) und das Aufzeichnen durch die Auswertungsergebnisaufzeichnungseinheit wiederholt durchgeführt werden, wenn die physikalische Eigenschaft des Lebensmittelprodukts durch die Einstellungseinheit (40) für die physische Eigenschaft des Lebensmittelprodukts geändert und eingestellt wird;
wobei die Speichereinheit (83) eine Lebensmittelprodukt-Prototypergebnis-Aufzeichnungseinheit aufweist, die eingerichtet ist, um ein Ergebnis einer experimentellen Herstellung aufzuzeichnen, die unter einer dementsprechend eingestellten Herstellungsbedingung durchgeführt wird, um die von dem Physikalische-Eigenschaft-Bestimmer (70) als geeignet bestimmte physikalische Eigenschaft aufzuweisen; und
die Vorrichtung ferner umfassend:
einen Herstellungsbedingungs-Bestimmer (84), der eingerichtet ist, um eine Herstellungsbedingung zu bestimmen, die eine physikalische Eigenschaft des Analyseobjekts als die von dem Physikalische-Eigenschaft-Bestimmer (70) als geeignet bestimmte physikalische Eigenschaft basierend auf einem Prototypergebnis, das in der Lebensmittelprodukt-Prototypergebnis-Aufzeichnungseinheit (83C) aufgezeichnet ist, einstellt, wobei die Ergebnisse der experimentellen Herstellung aufgezeichnet werden, wenn die Herstellungsbedingungen der experimentellen Herstellungen geändert werden;
wobei die Speichereinheit (83) eine Bestimmungs-Herstellungsbedingung-Aufzeichnungseinheit (83D) aufweist, die eingerichtet ist, um ein von dem Herstellungsbedingungs-Bestimmer (84) bestimmtes Ergebnis aufzuzeichnen.

2. Assistenzvorrichtung (200B) zur Lebensmittelproduktentwicklung nach Anspruch 1, wobei mindestens drei sich bewegende Wände in der Zunge (15) eingebettet sind.

3. Verfahren zur Lebensmittelproduktentwicklung, das von der Assistenzvorrichtung (200B) zur Lebensmittelproduktentwicklung für die Entwicklung eines einfach zu essenden oder einfach zu trinkenden Lebensmittelprodukts, eines medizinischen Produkts oder eines Quasi-Medikaments durchgeführt werden soll, das Verfahren umfassend:
einen Mundhöhlenmodellierungsschritt des Bildens eines zweidimensionalen Mundhöhlenmodells (11), das aus Mundhöhlenorganen in einem zweidimensionalen Raum unter Verwendung eines Teilchenverfahrens gebildet wird, wobei ein Verhalten eines Pseudo-Lebensmittelprodukts (41, 42, 43) in dem Mundhöhlenmodell (11) analysiert wird unter Verwendung von Organeigenschaften, Bewegungen der Mundhöhlenorgane und einer physikalischen Eigenschaft des Pseudo-Lebensmittelprodukts (41, 42, 43), die derart eingestellt sind, dass die Reproduktion der tatsächlichen Erscheinung des Schluckens erleichtert wird;
einen Mundhöhlenmodell(11)-Aufzeichnungsschritt des Aufzeichnens des Mundhöhlenmodells (11);
wobei das Mundhöhlenmodell (11) eine Mundhöhlenwand (12), eine Speiseröhre (13), Atemwege (14), eine Zunge (15), einen Gaumensegel (16) und einen Kehldeckel (17) enthält; wobei die Zunge (15), der Gaumensegel (16), der Kehldeckel (17) und ein Eingang der Speiseröhre (13) als elastische Körper eingestellt sind, während die anderen als starre Körper eingestellt sind, eine Bewegung der Zunge (15) durch eine Wellenbewegung ausgedrückt wird, Bewegungen des Gaumensegels (16) und des Kehldeckels (17) durch Hin- und Herbewegungen an den Basen und eine Rotationsbewegung um die Basen ausgedrückt werden und eine Bewegung eines Eingangsabschnitts der Speiseröhre (13) durch eine Hin- und Herbewegung in einer zu einer Zentralachse der Speiseröhre (13) senkrechten Richtung ausgedrückt wird, sich bewegende Wände in der Zunge (15) eingebettet sind und die Bewegung der Zunge (15) durchgeführt wird, während eine Schwingungsamplitude bei gleicher Phase und Phasenverschiebung geändert wird;
das Verfahren ferner umfassend:
einen Organeigenschaft-Einstellungsschritt des Einstellens einer Organeigenschaft jedes der Mundhöhlenorgane in dem Mundhöhlenmodell (11), das in dem Mundhöhlenmodell(11)-Aufzeichnungsschritt gespeichert wurde;
einen Organbewegung-Einstellungsschritt des Einstellens einer Bewegung jedes der Mundhöhlenorgane in dem Mundhöhlenmodell (11);
einen Einstellungsschritt einer physikalischen Eigenschaft für ein Lebensmittelprodukt des Einstellens eines Lebensmittelprodukts oder ähnlichen Produkts als ein Analyseobjekt oder eine physikalische Eigenschaft des Analyseobjekts;
einen Eingabeschritt des Eingebens eines Pseudo-Lebensmittelprodukts (41, 42, 43) in die Mundhöhle in den zweidimensionalen, wobei das Pseudo-Lebensmittelprodukt (41, 42, 43) durch Modellierung des Analyseobjekts gebildet wird;
einen Bewegungsanalyseschritt des Analysierens einer Bewegung jedes der Mundhöhlenorgane und eines Verhaltens des Pseudo-Lebensmittelprodukts (41, 42, 43), während es in dem Mundhöhlenmodell (11) unter Verwendung der Organeigenschaften, die in dem Organeigenschaft-Einstellungsschritt eingestellt wurden, der Bewegungen der Mundhöhlenorgane, die in dem Organbewegung-Einstellungsschritt eingestellt wurden, und der physikalischen Eigenschaft des Lebensmittelprodukts, die in dem Einstellungsschritt einer physikalischen Eigenschaft für ein Lebensmittelprodukt eingestellt wurde, und unter Verwendung des Teilchenverfahrens geschluckt wird;
einen Analyseergebnis-Aufzeichnungsschritt des Aufzeichnens eines Analyseergebnisses der Bewegung jedes der Mundhöhlenorgane und des Verhaltens des Pseudo-Lebensmittelprodukts (41, 42, 43);
einen Anzeigeschritt des Anzeigens auf einem virtuellen Bewegungsbildschirm, der in einem virtuellen Raum der Assistenzvorrichtung (200B) zur Lebensmittelproduktentwicklung gebildet ist, des Analyseergebnisses der Bewegung jedes der Mundhöhlenorgane und des Verhaltens des Pseudo-Lebensmittelprodukts (41, 42, 43), während es auf einem Bewegungsbildschirm geschluckt wird, wobei das Analyseergebnis in dem Bewegungsanalyseschritt analysiert wird;
einen Auswertungsschritt des Auswertens der Leichtigkeit des Essens oder der Leichtigkeit des Trinkens des Analyseobjekts basierend auf dem Verhalten des Pseudo-Lebensmittelprodukts (41, 42, 43), während es auf dem virtuellen Bewegungsbildschirm geschluckt wird, wobei der Auswertungsschritt automatisch auszuwertet, ob das Verhalten des Pseudo-Lebensmittelprodukts (41, 42, 43) auf dem virtuellen Bewegungsbildschirm mit einer vorbestimmten Bedingung übereinstimmt oder nicht, wobei die vorbestimmte Bedingung mindestens eine der folgenden Bedingungen ist: ob das Pseudo-Lebensmittelprodukt (41, 42, 43) in die Atemwege eintritt, ob das Pseudo-Lebensmittelprodukt (41, 42, 43) in der Speiseröhre (13) stecken bleibt, ob das Pseudo-Lebensmittelprodukt (41, 42, 43) an der Zunge (15) oder der Speiseröhre (13) klebt, ob ein Zeitraum von der Einführung des Pseudo-Lebensmittelprodukts (41, 42, 43) in die Mundhöhle bis zum Passieren dieses durch die Speiseröhre (13) innerhalb eines vorbestimmten Bereichs liegt, ob Druck, der durch das Pseudo-Lebensmittelprodukt (41, 42, 43) auf eine Wandfläche ausgeübt wird, kleiner gleich einem vorbestimmten Wert ist, ob Scherspannung, die durch das Pseudo-Lebensmittelprodukt (41, 42, 43) an der Wandfläche verursacht wird, kleiner gleich einem vorbestimmten Wert ist;
einen Auswertungsergebnis-Aufzeichnungsschritt des Aufzeichnens eines Auswertungsergebnisses der Leichtigkeit des Essens oder der Leichtigkeit des Trinkens des Analyseobjekts des Auswertungsschritts;
einen Physikalische-Eigenschaft-Bestimmungsschritt des Bestimmens der physikalischen Eigenschaft des Analyseobjekts, die basierend auf den Auswertungsergebnissen, die in dem Auswertungsergebnis-Aufzeichnungsschritt wiederholt aufgezeichnet wurden, als geeignet betrachtet wird, wobei der Einstellungsschritt der physikalischen Eigenschaft des Lebensmittelprodukts, der Bewegungsanalyseschritt, der Auswertungsschritt und der Auswertungsergebnis-Aufzeichnungsschritt wiederholt durchgeführt werden, wenn die physikalische Eigenschaft des Lebensmittelprodukts in dem Einstellungsschritt der physikalischen Eigenschaft des Lebensmittelprodukts geändert und eingestellt wird;
einen Lebensmittelproduktprototyp-Herstellungsschritt des Durchführens einer experimentellen Herstellung unter einer dementsprechend eingestellten Herstellungsbedingung, um die physikalische Eigenschaft aufzuweisen, die in dem Physikalische-Eigenschaft-Bestimmungsschritt als geeignet bestimmt wurde;
einen Lebensmittelproduktprototypergebnis-Aufzeichnungsschritt des Aufzeichnens eines Ergebnisses einer experimentellen Herstellung, die unter einer dementsprechend eingestellten Herstellungsbedingung durchgeführt wurde, um die physikalische Eigenschaft aufzuweisen, die durch den Physikalische-Eigenschaft-Bestimmer (70) als geeignet bestimmt wurde;
einen Herstellungsbedingung-Bestimmungsschritt des Bestimmens einer Herstellungsbedingung, die eine physikalische Eigenschaft des Analyseobjekts als die physikalische Eigenschaft einstellt, die in dem Physikalische-Eigenschaft-Bestimmungsschritt basierend auf dem Prototypergebnis in dem Lebensmittelproduktprototyp-Herstellungsschritt als geeignet bestimmt wurde, wobei die Ergebnisse der experimentellen Herstellung aufgezeichnet und verglichen werden, wenn die Herstellungsbedingungen der experimentellen Herstellungen geändert werden; und
einen Bestimmungs-Herstellungsbedingung-Aufzeichnungsschritt des Aufzeichnens eines Ergebnisses, das durch den Herstellungsbedingung-Bestimmungsschritt bestimmt wurde.

4. Verfahren zur Lebensmittelproduktentwicklung nach Anspruch 3, wobei mindestens drei sich bewegende Wände in der Zunge (15) eingebettet sind.

5. Verfahren zur Lebensmittelproduktentwicklung nach Anspruch 3 oder 4, wobei
das Verfahren wiederholt von dem Einstellungsschritt der physikalischen Eigenschaft des Lebensmittelprodukts bis zum Auswertungsschritt durchführt, während die physikalische Eigenschaft des Analyseobjekts in dem Einstellungsschritt der physikalischen Eigenschaft des Lebensmittelprodukts geändert und eingestellt wird.

6. Verfahren zur Lebensmittelproduktentwicklung nach einem der Ansprüche 3 bis 5, wobei
der Organeigenschaft-Einstellungsschritt eine Mundhöhlenwand (12) als einen starren Körper und eine Zunge (15) als einen elastischen Körper einstellt;
der Organbewegung-Einstellungsschritt eine Vielzahl von sich bewegenden Wänden in der Zunge (15) einstellt, wobei die Zunge (15) so eingestellt ist, dass sie sich durch Bewegen der Vielzahl von sich bewegenden Wänden in eine Richtung, die sich mit einer Oberfläche der Zunge (15) bei einem vorbestimmten Zeitraum und einem vorbestimmten Phasenunterschied überschneidet, in einer Wellenbewegung bewegt, und einen Gaumensegel (16), einen Kehldeckel (17) und eine Wand der Speiseröhre (13) so einstellt, dass sie sich zusammen bei einem vorbestimmten Phasenunterschied zur Wellenbewegung bewegen; und
der Bewegungsanalyseschritt die Zunge (15) und das Pseudo-Lebensmittelprodukt (41, 42, 43) als Teilchen behandelt.

7. Verfahren zur Lebensmittelproduktentwicklung nach einem der Ansprüche 3 bis 6, wobei
der Organbewegung-Einstellungsschritt eine Bewegung eines Gaumensegels (16) und eines Kehldeckels (17) als eine Bewegung eines Rotators einstellt, bei dem sich ein Rotationszentrum bewegt.

8. Verfahren zur Lebensmittelproduktentwicklung nach einem der Ansprüche 3 bis 7, wobei
der Einstellungsschritt der physikalischen Eigenschaft des Lebensmittelprodukts eine Vielzahl von flüssigen, halbfesten oder festen Pseudo-Lebensmittelprodukten (41, 42, 43) mit einer unterschiedlichen physikalischen Eigenschaft als ein Analyseobjekt einstellt; und
der Bewegungsanalyseschritt freie Flächen einer Vielzahl der Pseudo-Lebensmittelprodukten (41, 42, 43) und Grenzen zwischen der Vielzahl von Pseudo-Lebensmittelprodukten (41, 42, 43) bestimmt, wobei der Bewegungsanalyseschritt ein Übersetzungsverhalten der Vielzahl von Pseudo-Lebensmittelprodukten (41, 42, 43) analysiert.

9. Verfahren zur Lebensmittelproduktentwicklung nach einem der Ansprüche 3 bis 8, wobei
der Mundhöhlenmodellierungsschritt ein zweidimensionales Mundhöhlenmodell (11) bildet; und
der Bewegungsanalyseschritt das Verhalten des Pseudo-Lebensmittelprodukts (41, 42, 43) in einem zweidimensionalen Raum analysiert.

10. Verfahren zur Lebensmittelproduktentwicklung, umfassend die Schritte des Verfahrens nach einem der Ansprüche 3 bis 9; und
einen Lebensmittelproduktzubereitungsschritt des Zubereitens des Lebensmittelprodukts unter Verwendung einer Herstellungsbedingung, die in dem Herstellungsbedingung-Bestimmungsschritt als geeignet bestimmt wurde, um zu gestatten, dass das Lebensmittelprodukt die physikalische Eigenschaft aufweist, die als geeignet bestimmt wird.

## Revendications

1. Appareil d'aide au développement de produit alimentaire (200B) pour développer un produit alimentaire facile à manger ou facile à boire, un produit médicinal ou un quasi-médicament, comprenant :
une unité de modélisation de cavité buccale (10) configurée pour former un modèle de cavité buccale bidimensionnel (11) constitué d'organes de cavité buccale en utilisant un procédé de particules, dans lequel un comportement d'un pseudo-produit alimentaire (41, 42, 43) dans le modèle de cavité buccale (11) est analysé en utilisant les propriétés des organes, les mouvements des organes de cavité buccale et une propriété physique du pseudo-produit alimentaire (41, 42, 43) établis de sorte que la reproduction d'un phénomène réel de déglutition soit facilitée ;
une unité de mémorisation (83) configurée pour enregistrer le modèle de cavité buccale (11) ;
dans lequel le modèle de cavité buccale (11) comprend une paroi de cavité buccale (12), un gosier (13), un tractus respiratoire (14), une langue (15), un palais mou (16) et une épiglotte (17) ; la langue (15), le palais mou (16), l'épiglotte (17) et une entrée de gosier (13) sont établis en tant que corps élastiques alors que les autres sont établis en tant que corps rigides, un mouvement de la langue (15) est exprimé par un mouvement d'onde, les mouvements du palais mou (16) et de l'épiglotte (17) sont exprimés par des va-et-vient au niveau des bases et un mouvement de rotation autour des bases, et un mouvement d'une partie d'entrée du gosier (13) est exprimé par un va-et-vient dans une direction perpendiculaire à un axe central du gosier (13), des parois mobiles sont intégrées dans la langue (15) et le mouvement de la langue (15) est effectué tout en changeant une amplitude d'oscillation aux mêmes période et phase de décalage ;
l'appareil comprenant en outre :
une unité d'établissement de propriété d'organe (20) configurée pour établir une propriété d'organe de chacun des organes de cavité buccale dans le modèle de cavité buccale (11) mémorisé dans l'unité de mémorisation (83) ;
une unité d'établissement de mouvement d'organe (30) configurée pour établir un mouvement de chacun des organes de cavité buccale dans le modèle de cavité buccale (11) ;
une unité d'établissement de propriété physique de produit alimentaire (40) configurée pour établir un produit alimentaire, un produit médicinal ou un quasi-médicament en tant que cible d'analyse et une propriété physique de la cible d'analyse ;
une unité d'entrée (81) configurée pour entrer un pseudo-produit alimentaire (41, 42, 43) dans la cavité buccale dans le modèle de cavité buccale bidimensionnel (11), le pseudo-produit alimentaire (41, 42, 43) étant formé en modélisant la cible d'analyse ;
une unité d'analyse de mouvement (50) configurée pour analyser un mouvement de chacun des organes de cavité buccale et un comportement du pseudo-produit alimentaire (41, 42, 43) alors qu'il est avalé dans le modèle de cavité buccale (11) en utilisant les propriétés d'organe établies par l'unité d'établissement de propriété d'organe (20), les mouvements des organes de cavité buccale établis par l'unité d'établissement de mouvement d'organe (30), et la propriété physique du produit alimentaire établie par l'unité d'établissement de propriété physique de produit alimentaire (40) et en utilisant le procédé de particules ;
dans lequel l'unité de mémorisation (83) est configurée pour enregistrer un résultat d'analyse du mouvement de chacun des organes de cavité buccale et du comportement du pseudo-produit alimentaire (41, 42, 43) ;
l'appareil comprenant en outre :
une unité d'affichage (82A) configurée pour afficher, sur un écran mobile virtuel formé au niveau d'un espace virtuel de l'appareil d'aide au développement de produit alimentaire (200B), le résultat d'analyse du mouvement de chacun des organes de cavité buccale et du comportement du pseudo-produit alimentaire (41, 42, 43) alors qu'il est avalé sur un écran mobile, le résultat d'analyse étant analysé par l'unité d'analyse de mouvement (50) ;
une unité d'évaluation (60) configurée pour évaluer la facilité de consommation de nourriture et/ou la facilité de consommation de boisson de la cible d'analyse sur la base du comportement du pseudo-produit alimentaire (41, 42, 43) alors qu'il est avalé sur l'écran mobile virtuel, dans lequel l'unité d'évaluation (60) est conçue pour évaluer automatiquement si le comportement du pseudo-produit alimentaire (41, 42, 43) sur l'écran mobile virtuel satisfait à une condition prédéterminée ou non, dans lequel ladite condition prédéterminée est au moins l'une des conditions : si le pseudo-produit alimentaire (41, 42, 43) entre dans le tractus respiratoire, si le pseudo-produit alimentaire (41, 42, 43) reste bloqué dans le gosier (13), si le pseudo-produit alimentaire (41, 42, 43) adhère à la langue (15) ou au gosier (13), si une période de l'introduction du pseudo-produit alimentaire (41, 42, 43) dans la cavité buccale jusqu'à son passage à travers le gosier (13) est dans une plage prédéterminée, si une contrainte appliquée par le pseudo-produit alimentaire (41, 42, 43) à une surface de paroi est inférieure ou égale à une valeur prédéterminée, si une contrainte de cisaillement provoquée par le pseudo-produit alimentaire (41, 42, 43) au niveau de la surface de paroi est inférieure ou égale à une valeur prédéterminée ;
dans lequel l'unité de mémorisation (83) comporte une unité d'enregistrement de résultat d'évaluation (83B) configurée pour enregistrer un résultat d'évaluation de la facilité de consommation de nourriture ou de la facilité de consommation de boisson de la cible d'analyse évaluée par l'unité d'évaluation (60) ;
l'appareil comprenant en outre :
un dispositif de détermination de propriété physique (70) configuré pour déterminer la propriété physique de la cible d'analyse considérée comme appropriée sur la base des résultats d'évaluation enregistrés de manière répétée dans l'unité d'enregistrement de résultat d'évaluation (83B) ; dans lequel l'analyse par l'unité d'analyse de mouvement (50), l'évaluation par l'unité d'évaluation (60) et l'enregistrement par l'unité d'enregistrement de résultat d'évaluation sont effectués de manière répétée lorsque la propriété physique du produit alimentaire est changée et établie par l'unité d'établissement de propriété physique de produit alimentaire (40) ;
dans lequel l'unité de mémorisation (83) comporte une unité d'enregistrement de résultat de prototype de produit alimentaire configurée pour enregistrer un résultat d'une production expérimentale effectuée dans une condition de production établie de manière appropriée pour avoir la propriété physique déterminée comme étant appropriée par le dispositif de détermination de propriété physique (70) ; et
l'appareil comprenant en outre :
un dispositif de détermination de condition de production (84) configuré pour déterminer une condition de production qui établit une propriété physique de la cible d'analyse à la propriété physique déterminée comme étant appropriée par le dispositif de détermination de propriété physique (70) sur la base d'un résultat de prototype enregistré dans l'unité d'enregistrement de résultat de prototype de produit alimentaire (83C), dans lequel les résultats de production expérimentale sont enregistrés lorsque les conditions de production des productions expérimentales sont changées ;
dans lequel l'unité de mémorisation (83) comporte une unité d'enregistrement de détermination de condition de production (83D) configurée pour enregistrer un résultat déterminé par le dispositif de détermination de condition de production (84).

2. Appareil d'aide au développement de produit alimentaire (200B) selon la revendication 1, dans lequel au moins trois parois mobiles sont intégrées dans la langue (15).

3. Procédé de développement de produit alimentaire à effectuer par un appareil d'aide au développement de produit alimentaire (200B) pour développer un produit alimentaire facile à manger ou facile à boire, un produit médicinal ou un quasi-médicament, le procédé comprenant :
une étape de modélisation de cavité buccale pour former un modèle de cavité buccale bidimensionnel (11) constitué d'organes de cavité buccale dans un espace bidimensionnel en utilisant un procédé de particules, dans lequel un comportement d'un pseudo-produit alimentaire (41, 42, 43) dans le modèle de cavité buccale (11) est analysé en utilisant les propriétés des organes, les mouvements des organes de cavité buccale et une propriété physique du pseudo-produit alimentaire (41, 42, 43) établis de sorte que la reproduction d'un phénomène réel de déglutition soit facilitée ;
une étape d'enregistrement de modèle de cavité buccale (11) pour enregistrer le modèle de cavité buccale (11) ;
dans lequel le modèle de cavité buccale (11) comprend une paroi de cavité buccale (12), un gosier (13), un tractus respiratoire (14), une langue (15), un palais mou (16) et une épiglotte (17) ; la langue (15), le palais mou (16), l'épiglotte (17) et une entrée de gosier (13) sont établis en tant que corps élastiques alors que les autres sont établis en tant que corps rigides, un mouvement de la langue (15) est exprimé par un mouvement d'onde, les mouvements du palais mou (16) et de l'épiglotte (17) sont exprimés par des va-et-vient au niveau des bases et un mouvement de rotation autour des bases, et un mouvement d'une partie d'entrée du gosier (13) est exprimé par un va-et-vient dans une direction perpendiculaire à un axe central du gosier (13), des parois mobiles sont intégrées dans la langue (15) et le mouvement de la langue (15) est effectué tout en changeant une amplitude d'oscillation aux mêmes période et phase de décalage ;
le procédé comprenant en outre :
une étape d'établissement de propriété d'organe pour établir une propriété d'organe de chacun des organes de cavité buccale dans le modèle de cavité buccale (11) mémorisé à l'étape d'enregistrement du modèle de cavité buccale (11) ;
une étape d'établissement de mouvement d'organe pour établir un mouvement de chacun des organes de cavité buccale dans le modèle de cavité buccale (11) ;
une étape d'établissement de propriété physique de produit alimentaire pour établir un produit alimentaire ou un produit similaire en tant que cible d'analyse et une propriété physique de la cible d'analyse ;
une étape d'entrée pour entrer un pseudo-produit alimentaire (41, 42, 43) dans la cavité buccale dans le modèle bidimensionnel, le pseudo-produit alimentaire (41, 42, 43) étant formé en modélisant la cible d'analyse ;
une étape d'analyse de mouvement pour analyser un mouvement de chacun des organes de cavité buccale et un comportement du pseudo-produit alimentaire (41, 42, 43) alors qu'il est avalé dans le modèle de cavité buccale (11) en utilisant les propriétés d'organe établies à l'étape d'établissement de propriété d'organe, les mouvements des organes de cavité buccale établis à l'étape d'établissement de mouvement d'organe, et la propriété physique du produit alimentaire établie à l'étape d'établissement de propriété physique de produit alimentaire et en utilisant le procédé de particules ;
une étape d'enregistrement de résultat d'analyse pour enregistrer un résultat d'analyse du mouvement de chacun des organes de cavité buccale et du comportement du pseudo-produit alimentaire (41, 42, 43) ;
une étape d'affichage pour afficher, sur un écran mobile virtuel formé au niveau d'un espace virtuel de l'appareil d'aide au développement de produit alimentaire (200B), le résultat d'analyse du mouvement de chacun des organes de cavité buccale et du comportement du pseudo-produit alimentaire (41, 42, 43) alors qu'il est avalé sur un écran mobile, le résultat d'analyse étant analysé à l'étape d'analyse de mouvement ;
une étape d'évaluation pour évaluer la facilité de consommation de nourriture ou la facilité de consommation de boisson de la cible d'analyse sur la base du comportement du pseudo-produit alimentaire (41, 42, 43) alors qu'il est avalé sur l'écran mobile virtuel, dans lequel l'étape d'évaluation évalue automatiquement si le comportement du pseudo-produit alimentaire (41, 42, 43) sur l'écran mobile virtuel satisfait à une condition prédéterminée ou non, dans lequel ladite condition prédéterminée est au moins l'une des conditions : si le pseudo-produit alimentaire (41, 42, 43) entre dans le tractus respiratoire, si le pseudo-produit alimentaire (41, 42, 43) est bloqué dans le gosier (13), si le pseudo-produit alimentaire (41, 42, 43) adhère à la langue (15) ou au gosier (13), si une période de l'introduction du pseudo-produit alimentaire (41, 42, 43) dans la cavité buccale jusqu'à son passage à travers le gosier (13) est dans une plage prédéterminée, si la contrainte appliquée par le pseudo-produit alimentaire (41, 42, 43) à une surface de paroi est inférieure ou égale à une valeur prédéterminée, si la contrainte de cisaillement provoquée par le pseudo-produit alimentaire (41, 42, 43) au niveau de la surface de paroi est inférieure ou égale à une valeur prédéterminée ;
une étape d'enregistrement de résultat d'évaluation pour enregistrer un résultat d'évaluation de la facilité de consommation de nourriture ou de la facilité de consommation de boisson de la cible d'analyse de ladite étape d'évaluation ;
une étape de détermination de propriété physique pour déterminer la propriété physique de la cible d'analyse considérée comme appropriée sur la base des résultats d'évaluation enregistrés de manière répétée à l'étape d'enregistrement de résultat d'évaluation, dans lequel l'étape d'établissement de propriété physique de produit alimentaire, l'étape d'analyse de mouvement, l'étape d'évaluation et l'étape d'enregistrement de résultat d'évaluation sont effectuées de manière répétée lorsque la propriété physique du produit alimentaire est changée et établie à l'étape d'établissement de propriété physique de produit alimentaire ;
une étape de production de prototype de produit alimentaire pour effectuer une production expérimentale dans une condition de production établie de manière appropriée pour avoir la propriété physique déterminée comme étant appropriée à l'étape de détermination de propriété physique ;
une étape d'enregistrement de résultat de prototype de produit alimentaire pour enregistrer un résultat d'une production expérimentale effectuée dans une condition de production établie de manière appropriée pour avoir la propriété physique déterminée comme étant appropriée par le dispositif de détermination de propriété physique (70) ;
une étape de détermination de condition de production pour déterminer une condition de production qui établit une propriété physique de la cible d'analyse à la propriété physique déterminée comme étant appropriée à l'étape de détermination de propriété physique sur la base du résultat de prototype à l'étape de production de prototype de produit alimentaire, dans lequel les résultats de production expérimentale sont enregistrés et comparés lorsque les conditions de production des productions expérimentales sont changées ; et
une étape d'enregistrement de détermination de condition de production pour enregistrer un résultat déterminé par l'étape de détermination de condition de production.

4. Procédé de développement de produit alimentaire selon la revendication 3, dans lequel au moins trois parois mobiles sont intégrées dans la langue (15).

5. Procédé de développement de produit alimentaire selon la revendication 3 ou 4, dans lequel
le procédé effectue de manière répétée les étapes de l'étape d'établissement de propriété physique de produit alimentaire à l'étape d'évaluation alors que la propriété physique de la cible d'analyse est changée et établie à l'étape d'établissement de propriété physique de produit alimentaire.

6. Procédé de développement de produit alimentaire selon l'une des revendications 3 à 5, dans lequel
l'étape d'établissement de propriété d'organe établit une paroi de cavité buccale (12) en tant que corps rigide et une langue (15) en tant que corps élastique ;
l'étape d'établissement de mouvement d'organe établit une pluralité de parois mobiles dans la langue (15), la langue (15) étant établie de manière à bouger en un mouvement d'onde en déplaçant la pluralité de parois mobiles dans une direction croisant une surface de la langue (15) avec une période prédéterminée et une différence de phase prédéterminée, et établit un palais mou (16), une épiglotte (17), et une paroi de gosier (13) de manière à ce qu'ils bougent ensemble avec une différence de phase prédéterminée par rapport au mouvement d'onde ; et
l'étape d'analyse de mouvement traite la langue (15) et le pseudo-produit alimentaire (41, 42, 43) en tant que particules.

7. Procédé de développement de produit alimentaire selon l'une des revendications 3 à 6, dans lequel
l'étape d'établissement de mouvement d'organe établit un mouvement d'un palais mou (16) et d'une épiglotte (17) en tant que mouvement d'un rotateur où un centre de rotation se déplace.

8. Procédé de développement de produit alimentaire selon l'une des revendications 3 à 7, dans lequel
l'étape d'établissement de propriété physique de produit alimentaire établit une pluralité de pseudo-produits alimentaires liquides, semi-solides, ou solides (41, 42, 43) avec une propriété physique différente en tant que cible d'analyse ; et
l'étape d'analyse de mouvement détermine les surfaces libres d'une pluralité des pseudo-produits alimentaires (41, 42, 43) et les frontières entre la pluralité de pseudo-produits alimentaires (41, 42, 43), l'étape d'analyse de mouvement analysant un comportement d'engrènement de la pluralité de pseudo-produits alimentaires (41, 42, 43).

9. Procédé de développement de produit alimentaire selon l'une des revendications 3 à 8, dans lequel
l'étape de modélisation de cavité buccale forme un modèle de cavité buccale bidimensionnel (11) ; et
l'étape d'analyse de mouvement analyse le comportement du pseudo-produit alimentaire (41, 42, 43) dans un espace bidimensionnel.

10. Procédé de production de produit alimentaire, comprenant les étapes du procédé selon l'une des revendications 3 à 9 ; et
une étape de préparation de produit alimentaire pour préparer le produit alimentaire en utilisant une condition de production déterminée comme étant appropriée à l'étape de détermination de condition de production pour permettre que le produit alimentaire ait la propriété physique qui est déterminée comme étant appropriée.
